# EUROPEAN PATENT APPLICATION

(11) **EP 1 655 289 A1**
(43) Date of publication of application: **10.05.2006**
(21) Application number: 04026190.1
(22) Date of filing: 04.11.2004
(51) Int. Cl.: C07D 239/78, A61K 31/505, A61P 35/00

(54) **Quinazoline derivatives, process for their preparation, their use as antimitotics and pharmaceutical compositions comprising said derivatives**

(71) Applicant: EMBL, D-69117 Heidelberg (DE); Universität Leipzig, D-04109 Leipzig (DE); Max-Planck-Gesellschaft zur Förderung der Wissenschaften e.V., 80539 München (DE)
(72) Inventor: Surrey, Thomas, 69115 Heidelberg (DE); Athanassios, Giannis, 04316 Leipzig (DE); Vernos, Isabelle, 69115 Heidelberg (DE); Gartner, Michael, 76327 Pfinztal (DE); Sunder-Plassmann, Nils, 55234 Wendelsheim (DE); Vasiliki, Sarli, 04317 Leipzig (DE); Mayer, Thomas, 81375 München (DE); Huemmer, Stefan, 97493 Bergrheinfeld (DE)
(74) Representative: Reitstötter - Kinzebach

(57) **Abstract**

The invention relates to quinazoline derivatives of formula (I) 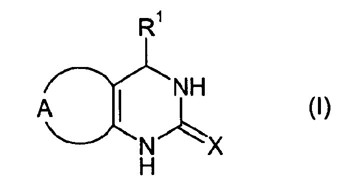 in which R¹, X and A have the meanings as indicated, or to a pharmaceutically acceptable salt thereof, e.g.

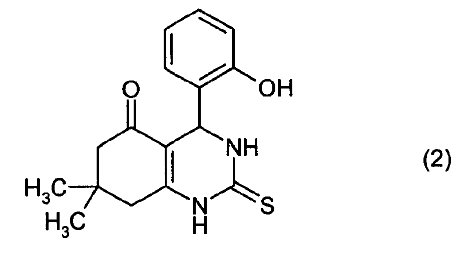

Such compounds are useful as modulators, in particular inhibitors, of the mitotic kinesin Eg5. i.e. the human protein (also called KSP), the *Xenopus laevis* homologue, further homologues from other species as well as allelic variants and functionally related proteins.

The invention also relates to processes for their preparation, pharmaceutical compositions comprising said quinazoline derivatives, and the use of said quinazoline derivatives, in particular for treating diseases such as cancer.

## Description

The present invention relates to quinazoline derivatives, processes for their preparation, pharmaceutical compositions comprising said quinazoline derivatives, and the use of said quinazoline derivatives as antimitotics and thus for treating diseases such as cancer.

Anti-cancer drugs that perturb mitosis, for example vinca alkaloids, taxol and epothilone, play a major role in the therapy of malignant diseases. One of their major drawbacks is the fact that they are all directed against the same protein, tubulin, the subunit of microtubules that form the mitotic spindle. However, microtubules are also involved in many other cellular processes such as maintenance of organelles, cell shape, cell motility, synaptic vesicles and intracellular transport phenomena. Interference with their formation or depolymerisation often leads to dose-limiting toxic side-effects.

The discovery of a new class of proteins, the mitotic kinesins, allows a novel approach to cancer treatment. These proteins are exclusively involved in the formation and function of the mitotic spindle and some of them are only expressed in proliferating cells. Their inhibition leads to cell cycle arrest and ultimately to apoptosis without interfering with other microtubule-dependent processes.

The mitotic kinesin Eg5 (the *Xenopus laevis* analogue of human kinesin spindle protein, KSP) plays an important role in the early stages of mitosis. It mediates centrosome separation and formation of the bipolar mitotic spindle. Inhibition of Eg5 leads to cell cycle arrest during mitosis and cells with a monopolar spindle, so called monoasters. In 1999 monastrol, 4-(3-hydroxyphenyl)-5-ethoxycarbonyl-6-methyl-3,4-dihydropyrimidin-2(1*H*), was identified as the first small-molecule inhibitor of Eg5 by screening a large library of synthetic compounds (Mayer, T. U., Kapoor, T. M., Haggarty, S. J., King, R. W., Schreiber, S. L., and Mitchison, T. J. (1999). Small Molecule Inhibitor of Mitotic Spindle Bipolarity Identified in a Phenotype-Based Screen. Science 289, 971-974). Monastrol is an allosteric inhibitor of the mitotic kinesin Eg5 (Maliga, Z., Kapoor, T.M. and Mitchison, T.J. (2002). Evidence that Monastrol is an allosteric inhibitor of the mitotic kinesin Eg5. Chemistry & Biology 9, 989-996) that binds some 12 A away from the nucleotide binding site of the protein. In doing so, monastrol triggers both local and distal structural changes throughout the motor domain (Yan, Y., Sardana, V., Xu, B., Homnick, C., Halczenko, W., Buser, C. A., Schaber, M., Hartman, G. D., Huber, H. E., and Lawrence, C. K. (2004). Inhibition of a Mitotic Motor Protein: Where, How, and Conformational Consequences. J. Mol. Biol. 335, 547-554). In the meantime, four other inhibitors of Eg5 have been published: Terpendole E, S-trityl-L-cysteine, HR22C16 and CK0106023 and further quinazoline-4-on derivatives (Nakazawa, J., Yajima, J., Usui, T., Ueki, M., Takatsuki, A., Imoto, M., Toyoshima, Y. Y., and Osada, H. (2003). A Novel Action of Terpendole E on the Motor Activity of Mitotic Kinesin Eg5. Chem. Biol. *10*, 131-137; DeBonis, S., Skoufias, D. A., Lebeau, L., Lopez, R., Robin, G., Margolis, R. L., Wade, R. H., and Kozielski, F. (2004). *In vitro* screening for inhibitors of the human mitotic kinesin Eg5 with antimitotic and antitumor activities. Mol. Cancer. Ther. 3, 1079-1090); Hotha, S., Yarrow, J. C., Yang, J. G., Garrett, S., Renduchintala, K. V., Mayer, T. U., and Kapoor, T. M. (2003). HR22C16: A Potent Small-Molecule Probe for the Dynamics of Cell Division. Angew. Chem. *115*, 2481-2484; Sakowicz, R., Finer, J. T., Beraud, C., Crompton, A., Lewis, E., Fritsch, A., Lee, Y., Mak, J., Moody, R., Turincio, R., Chabala, J. C., Gonzales, P., Roth, S., Weitman, S., and Wood, K. W. (2004). Antitumor Activity of a Kinesin Inhibitor. Cancer Res. *64,* 3276-3280; WO-A 01/98278). WO-A 02/078639 describes further Eg5 inhibitors which are structurally similar to Monastrol.

It was an object of the present invention to provide further compounds which are useful as antimitotics.

Surprisingly, it was found that certain quinazoline derivatives are effective compounds for the inhibition of Eg5 *in vitro* and for arresting cultured cells in mitosis.

The present invention thus relates to quinazoline derivatives of formula (I) 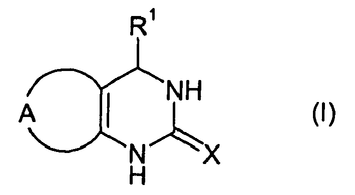 in which
- R¹: denotes phenyl or six-membered heteroaryl, optionally substituted with one to five identical or different substituents selected from the group consisting of halogen, cyano, nitro, hydroxyl, mercapto, C₁-C₆-alkyl, C₁-C₆-alkoxy, C₁-C₆₋alkylthio, C₁-C₆-haloalkyl, C₁-C₆-haloalkoxy, carboxyl, C₁-C₆-alkylcarbonyl, C₁₋C₆-alkoxycarbonyl and C₁-C₆-alkylcarbonyloxy;
- X: denotes S or O;
- A: together with the carbon atoms to which it is bonded forms a five-, six- or seven-membered, partially unsaturated or aromatic, homocyclic or heterocyclic ring which may carry up to ten identical or different substituents selected from the group consisting of halogen, cyano, nitro, hydroxyl, oxo, mercapto, thio, amino, C₁-C₆-alkyl, C₁-C₆-alkoxy, C₁-C₆-alkylthio, C₁-C₆₋alkylamino, di-(C₁-C₆-alkyl)amino, C₁-C₆-haloalkyl, C₁-C₆-haloalkoxy, carboxyl, carbamoyl, C₁-C₆-alkylcarbonyl, C₁-C₆-alkoxycarbonyl, C₁-C₆₋alkylcarbonyloxy, C₁-C₆-alkylaminocarbonyl, di-(C₁-C₆-alkyl)amincarbonyl, C₁-C₆-alkylsulfinyl, C₁-C₆-alkylsulfonyl, hydroxysulfonyl, aminosulfonyl, C₁-C₆₋alkylaminosulfonyl and di-(C₁-C₆-alkyl)aminosulfonyl,
or a pharmaceutically acceptable salt thereof.

In the symbol definitions given in formula (I) above, collective terms are used which generally represent the following substituents:
- halogen: fluorine, chlorine, bromine or iodine;
- C₁-C₆-alkyl and the alkyl moieties of C₁-C₆-alkoxy, C₁-C₆-alkylthio, C₁-C₆₋alkylcarbonyl, C₁-C₆-alkoxycarbonyl, C₁-C₆-alkylcarbonyloxy, C₁-C₆-alkylamino, di-(C₁₋C₆-alkyl)amino, C₁-C₆-alkylaminocarbonyl, di-(C₁-C₆-alkyl)aminocarbonyl, C₁-C₆₋alkylsulfinyl, C₁-C₆-alkylsulfonyl, C₁-C₆-alkylaminosulfonyl, di-(C₁-C₆₋alkyl)aminosulfonyl: saturated, straight-chain or branched hydrocarbon radicals having 1 to 6 carbon atoms, especially 1 to 4 carbon atoms, such as methyl, ethyl, propyl, 1-methylethyl, butyl, 1-methylpropyl, 2-methylpropyl, 1,1-dimethylethyl, or pentyl, 1-methylbutyl, 2-methylbutyl, 3-methylbutyl, 2,2-di-methylpropyl, 1-ethylpropyl, hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl and 1-ethyl-2-methylpropyl;
- C₁-C₆-haloalkyl and the haloalkyl moieties of C₁-C₆-haloalkoxy: straight-chain or branched alkyl groups having 1 to 6 carbon atoms, especially 1 to 4 carbon atoms (as mentioned above), where the hydrogen atoms in these groups may be partially or fully replaced by halogen atoms as mentioned above, for example C₁-C₂-haloalkyl, such as chloromethyl, bromomethyl, dichloromethyl, trichloromethyl, fluoromethyl, difluoromethyl, trifluoromethyl, chlorofluoromethyl, dichlorofluoromethyl, chlorodifluoromethyl, 1-chloroethyl, 1-bromoethyl, 1-fluoroethyl, 2-fluoroethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl and pentafluoroethyl;
- 5-, 6- or 7-membered homoocyclic ring: mono- or bicyclic hydrocarbon rings; unsaturated includes partially unsaturated, e.g. mono-unsaturated, and aromatic; said homocycles in particular include:

- 6-membered aromatic rings (aryl), such as benzene (phenyl);
- 5-membered carbocyclic rings (carbocyclyl), such as cyclopenten(yl), cyclopent-1,3-dien(yl);
- 6-membered carbocyclic rings (carbocyclyl), such as cyclohexen(yl), cyclohex-1,3-dien(yl) and cyclohex-1,4-dien(yl);
- 7-membered carbocyclic rings(carbocyclyl), such as cyclohepten(yl), cyclohept-1,3-dien(yl), cyclohept-1,4-dien(yl), cyclohept-1,5-dien(yl) and cyclohept-1,3,5-trien(yl);
- 5-, 6- or 7-membered heterocyclic ring: mono- or bicyclic hydrocarbon rings containing one to four heteroatoms selected from the group consisting of a nitrogen atom, an oxygen atom and a sulfur atom; unsaturated includes partially unsaturated, e.g. mono-unsaturated, and aromatic; said heterocyclic rings in particular include:

- 5-membered aromatic heterocyclic rings (heteroaryl), containing one to four nitrogen atoms or one to three nitrogen atoms and one sulfur or oxygen atom: 5-membered heteroaryl groups which, in addition to carbon atoms, may contain one to four nitrogen atoms or one to three nitrogen atoms and one sulfur or oxygen atom as ring members, for example, furan(-2-yl), furan(-3-yl), thiophen(-2-yl), thiophen(-3-yl), pyrrol(-2-yl), pyrrol(-3-yl), isoxazol(-3-yl)l, isoxazol(-4-yl), isoxazol(-5-yl)l, isothiazol(-3-yl), isothiazol(-4-yl), isothiazol(-5-yl)l, pyrazol(-3-yl), pyrazol(-4-yl), pyrazol(-5-yl), oxazol(-2-yl), oxazol(-4-yl), oxazol(-5-yl), thiazol(-2-yl), thiazol(-4-yl), thiazol(-5-yl), imidazol(-2-yl), imidazol(-4-yl), 1,2,4-oxadiazol(-3-yl), 1,2,4-oxadiazol(-5-yl), 1,2,4-thiadiazol(-3-yl), 1,2,4-thiadiazol(-5-yl), 1,2,3-triazol(-2-yl), 1,2,4-triazol(-3-yl), tetrazol(yl), 1,3,4-oxadiazol(-2-yl), 1,3,4-thiadiazol(-2-yl) and 1,3,4-triazol(-2-yl);
- 6-membered aromatic heterocyclic rings (heteroaryl), containing one to four nitrogen atoms: 6-membered heteroaryl groups which, in addition to carbon atoms, may contain one to three or one to four nitrogen atoms as ring members, for example, pyridin(-2-yl), pyridin(-3-yl), pyridin(-4-yl), pyridazin(-3-yl), pyridazin(-4-yl), pyrimidin(-2-yl), pyrimidin(-4-yl), pyrimidin(-5-yl), pyrazin(-2-yl), 1,2,3-triazin(yl), 1,3,5-triazin(-2-yl) and 1,2,4-triazin(-3-yl);
- 5- and 6-membered heterocyclic rings (heterocyclyl), containing one to four nitrogen atoms or one to three nitrogen atoms and one sulfur or oxygen atom: for example, pyrazolidin(-3-yl), pyrazolidin(-4-yl), pyrazolidin(-5-yl), 2-pyrrolodin(-2-yl), 2-pyrrolodin(-3-yl), 3-pyrrolodin(-2-yl), 3-pyrrolodin(-3-yl), piperidin(-1-yl), piperidin(-2-yl), piperidin(-3-yl), piperidin(-4-yl), pyridin(1,2-dihydro)-2-on(-1-yl), piperazin(-2-yl), morpholin(-4-yl), thiomorpholin(-4-yl).

In the compounds of formula (I), R¹ represents optionally substituted phenyl or optionally substituted 6-membered heteroaryl. The phenyl or heteroaryl group may carry up to 5, preferably up to 3, identical or different substituents. According to a particular embodiment, the phenyl of heteroaryl group carries at least one substituent which is in the ortho or meta position. Particular preference is given to monosubstituted aryl or heteroaryl groups.

The substituent(s) on the phenyl or heteroaryl group are selected from the group consisting of halogen, cyano, nitro, hydroxyl, mercapto, C₁-C₆-alkyl, C₁-C₆-alkoxy, C₁₋C₆-alkylthio, C₁-C₆-haloalkyl, C₁-C₆-haloalkoxy, carboxyl, C₁-C₆-alkylcarbonyl, C₁-C₆₋alkoxycarbonyl and C₁-C₆-alkylcarbonyloxy. Particular preference is given to substituents selected from the group consisting or halogen, cyano, nitro, hydroxyl, mercapto, methyl, methoxy, methylthio, trifluoromethyl, trifluoromethoxy, carboxyl, acetyl, methoxycarbonyl and methoxycarbonyloxy. Even more preferred substituents are selected from the group consisting of halogen, in particular fluoro or nitro, and hydroxyl.

According to a particular embodiment, the present invention relates to quinazoline derivatives of formula (I) wherein R¹ represents a substituted phenyl group. Accordingly, R¹ in formula (I) denotes a group of formula (II): 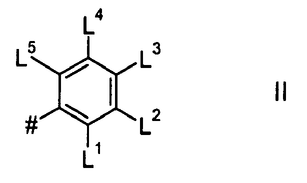 wherein L¹ to L⁵ independently denote halogen, cyano, nitro, hydroxyl, mercapto, C₁₋C₆-alkyl, C₁-C₆-alkoxy, C₁-C₆-alkylthio, C₁-C₆-haloalkyl, C₁-C₆-haloalkoxy, carboxyl C₁₋C₆-alkylcarbonyl, C₁-C₆-alkoxycarbonyl, C₁-C₆-alkylcarbonyloxy, and # marks the bond to the quinazoline nucleus, wherein at least one of L¹ to L⁵ is not hydrogen.

Particular preference is given to compounds wherein in formula (II) at least one of L¹ and L² is not hydrogen. Especially preferred are compounds, wherein in formula (I) R¹ denotes a group or formula (IIa): 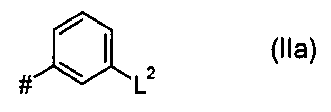 wherein L² is as defined above.

According to a preferred embodiment, the present invention relates to quinazoline derivatives of formula (I), wherein R¹ is a 3-hydroxyphenyl group.

In the compounds of formula (I), X can be a sulfur atom or an oxygen atom. The present invention thus relates to quinazoline-2(1*H*)-thione (X=S) or quinazoline-2 (1*H*)-one (X=O) derivatives. Particular preference is given to the thione derivatives.

In the compounds of formula (I), A forms a ring that is fused to the 3,4-dihydropyrimidine-2(1*H*)-one or 3,4-dihydropyrimidine-2(1*H*)-thione moiety. While said fused ring can be a 5-, 6- or 7-membered ring, particular preference is given to compounds of formula (I) wherein the fused ring is 6-membered (quinazolines). According to a particular embodiment, the present invention thus relates to quinazoline derivatives of formulal (Ia): 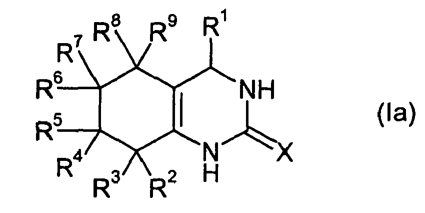 wherein R¹ and X are as defined above, and R², R³, R⁴, R⁵, R⁶, R⁷, R⁸, R⁹ independently denote hydrogen, halogen, cyano, nitro, hydroxyl, oxo, mercapto, thio, amino, C₁-C₆-alkyl, C₁-C₆-alkoxy, C₁-C₆-alkylthio, C₁-C₆-alkylamino, di-(C₁-C₆₋alkyl)amino, C₁-C₆-haloalkyl, C₁-C₆-haloalkoxy, carboxyl, carbamoyl, C₁-C₆₋alkylcarbonyl, C₁-C₆-alkoxycarbonyl, C₁-C₆-alkylcarbonyloxy, C₁-C₆₋alkylaminocarbonyl, di-(C₁-C₆-alkyl)amincarbonyl, C₁-C₆-alkylsulfinyl, C₁-C₆₋alkylsulfonyl, hydroxysulfonyl, aminosulfonyl, C₁-C₆-alkylaminosulfonyl or di-(C₁-C₆₋alkyl)aminosulfonyl, or R³ together with R⁵, R⁵ together with R⁷ and/or R⁷ together with R⁹ form a bond, or R² together with R³, R⁴ together with R⁵, R⁶ together with R⁷, and/or R⁸ together with R⁹ form an oxo or thio group.

Due to the double bond of the 3,4-dihydropyrimidine moiety the fused ring is unsaturated. This includes partially unsaturated, in particular mono-unsaturated, and aromatic rings.

According to a particular embodiment, the present invention relates to compounds of formula (Ia), wherein R⁸ together with R⁹ form an oxo group, i.e. 5-oxo-3,4,5,6,7,8-hexahydro-quinazoline-2(1*H*)-thione or 5-oxo-3,4,5,6,7,8-hexahydro-quinazoline-2(1*H*)-one derivatives of the following formula (Ib): 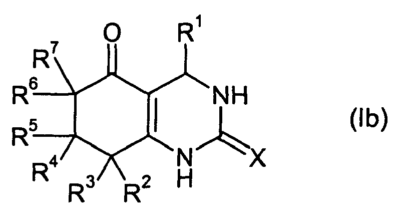 wherein R¹ and X are as defined above, and R², R³, R⁴, R⁵, R⁶, R⁷ independently denote hydrogen, halogen, cyano, nitro, hydroxyl, oxo, mercapto, thio, amino, C₁-C₆₋alkyl, C₁-C₆-alkoxy, C₁-C₆-alkylthio, C₁-C₆-alkylamino, di-(C₁-C₆-alkyl)amino, C₁-C₆₋haloalkyl, C₁-C₆-haloalkoxy, carboxyl, carbamoyl, C₁-C₆-alkylcarbonyl, C₁-C₆₋alkoxycarbonyl, C₁-C₆-alkylcarbonyloxy, C₁-C₆-alkylaminocarbonyl, di-(C₁-C₆₋alkyl)amincarbonyl, C₁-C₆-alkylsulfinyl, C₁-C₆-alkylsulfonyl, hydroxysulfonyl, aminosulfonyl, C₁-C₆-alkylaminosulfonyl or di-(C₁-C₆-alkyl)aminosulfonyl, or R³ together with R⁵ or R⁵ together with R⁷ form a bond, or R² together with R³, R⁴ together with R⁵, and/or R⁶ together with R⁷ form an oxo or thio group.

According to a further particular embodiment, the present invention relates to compounds of formula (Ia), wherein R⁸ is hydrogen and R⁹ is hydroxyl, i.e. 5-hydroxy-3,4,5,6,7,8-hexahydro-quinazoline-2(1*H*)-thione or 5-hydroxy-3,4,5,6,7,8-hexahydroquinazoline-2(1*H*)-one derivatives of the following formula (Ic): 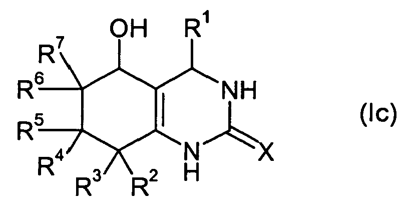 wherein R¹ and X are as defined above, and R², R³, R⁴, R⁵, R⁶, R⁷ independently denote hydrogen, halogen, cyano, nitro, hydroxyl, oxo, mercapto, thio, amino, C₁-C₆₋alkyl, C₁-C₆-alkoxy, C₁-C₆-alkylthio, C₁-C₆-alkylamino, di-(C₁-C₆-alkyl)amino, C₁-C₆₋haloalkyl, C₁-C₆-haloalkoxy, carboxyl, carbamoyl, C₁-C₆-alkylcarbonyl, C₁-C₆₋alkoxycarbonyl, C₁-C₆-alkylcarbonyloxy, C₁-C₆-alkylaminocarbonyl, di-(C₁-C₆₋alkyl)amincarbonyl, C₁-C₆-alkylsulfinyl, C₁-C₆-alkylsulfonyl, hydroxysulfonyl, aminosulfonyl, C₁-C₆-alkylaminosulfonyl or di-(C₁-C₆-alkyl)aminosulfonyl, or R³ together with R⁵ or R⁵ together with R⁷ form a bond, or R² together with R³, R⁴ together with R⁵, and/or R⁶ together with R⁷ form an oxo or thio group.

According to a further particular embodiment, the present invention relates to compounds of formula (Ia), wherein R³ together with R⁵ and R⁷ together with R⁹ form a bond, i.e. 3,4-dihydro-quinazoline-2(1*H*)-thione or 3,4-dihydro-quinazoline-2(1*H*)-one derivatives of the following formula (Id): 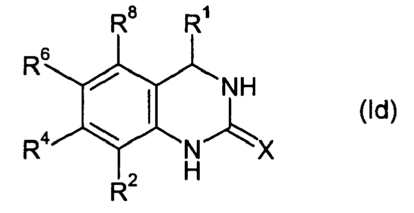 wherein R¹ and X are as defined above, and R², R⁴, R⁶, R⁸ independently denote hydrogen, halogen, cyano, nitro, hydroxyl, mercapto, amino, C₁-C₆-alkyl, C₁-C₆-alkoxy, C₁-C₆-alkylthio, C₁-C₆-alkylamino, di-(C₁-C₆-alkyl)amino, C₁-C₆-haloalkyl, C₁-C₆₋haloalkoxy, carboxyl, carbamoyl, C₁-C₆-alkylcarbonyl, C₁-C₆-alkoxycarbonyl, C₁-C₆₋alkylcarbonyloxy, C₁-C₆-alkylaminocarbonyl, di-(C₁-C₆-alkyl)amincarbonyl, C₁-C₆₋alkylsulfinyl, C₁-C₆-alkylsulfonyl, hydroxysulfonyl, aminosulfonyl, C₁-C₆₋alkylaminosulfonyl or di-(C₁-C₆-alkyl)aminosulfonyl.

In addition to the above-described compounds of the formulae (Ia), (Ib), (Ic) and (Id), in which A forms a homocyclic ring, the present invention also relates to quinazoline derivatives of formula (I), wherein A together with the carbon atoms to which it is bonded forms a heterocyclic ring. These include, for instance, compounds of the following formula (Ie): 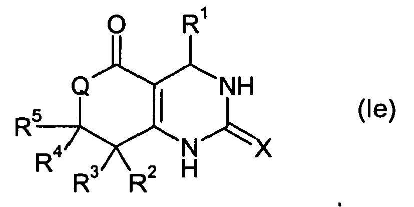 wherein Q is O or NH and R¹, R², R³, R⁴, R⁵, and X are as defined above.

In the compounds of formula (I), the fused ring may carry up to ten identical or different substituents. Particular preference is given to the above substituents wherein the alkyl moieties have 1 to 3 carbon atoms. In particular said substituents are selected from the group consisting of halogen, cyano, nitro, hydroxyl, oxo, mercapto, thio, amino, methyl, methoxy, methylthio, methylamino, dimethylamino, trifluoromethyl, trifluoromethoxy, carboxyl, carbamoyl, acetyl, methoxycarbonyl, methylcarbonyloxy, methylaminocarbonyl, dimethylaminocarbonyl, methylsulfinyl, methylsulfonyl, hydroxysulfonyl, aminosulfonyl, methylaminosulfonyl and dimethylaminosulfonyl. Even more preferred are substituents selected from the group consisting of halogen, in particuler fluoro, chloro or bromo, methyl and methoxy.

According to a particular embodiment, the present invention relates to compounds of formula (Ia), (Ib), (Ic) or (Ie), wherein at least one of R², R³, R⁴ and R⁵ is different from hydrogen. Particular preference is given to compounds of formula (Ib), (Ic) or (Ie), wherein R² and R³ are hydrogen, and R⁴ and R⁵ are both methyl.

According to a further particular embodiment, the present invention relates to compounds of formula (Id), wherein at least one of R², R⁵ and R⁶ is different from hydrogen. Particular preference is given to compounds of formula (Id), wherein R² is hydrogen, methyl or methoxy, R⁵ is hydrogen or halogen, in particular fluoro or chloro, and R⁶ is hydrogen or halogen, in particular chloro or bromo.

Most preferred are the compounds compiled in the following table 1:

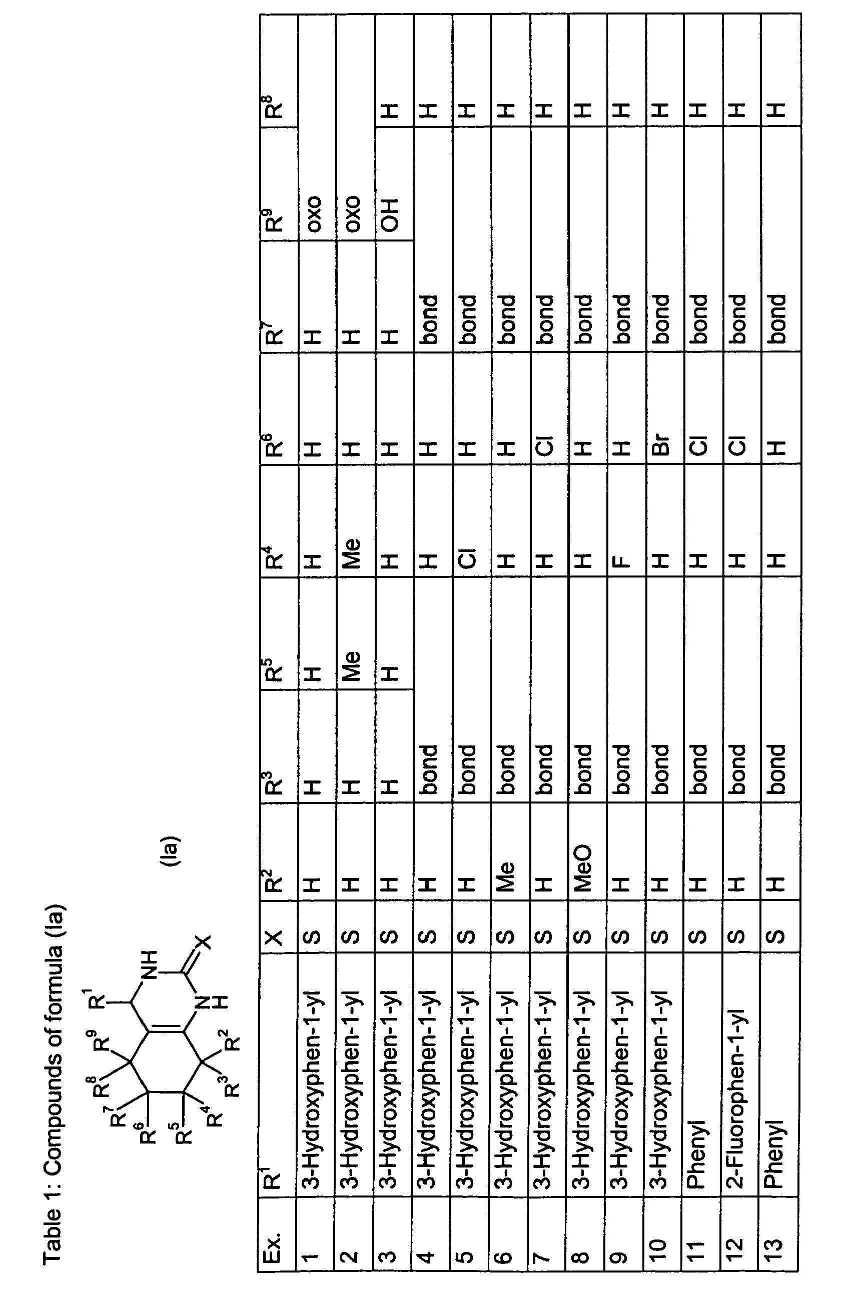

Salts of the compounds of the present invention include acid addition and base addition salts which are preferably formed with pharmaceutically acceptable acids or bases. Particular preference is given to salts formed with inorganic or organic acids, such as hydrochloric, hydrobromic and hydroiodic acid, phosphoric acid, sulfuric acid, nitric acid, p-toluenesulfonic acid, methenesulfonic acid, formic acid, acidic acid, propionic acid, citric acid, tartaric acid, succinic acid, benzoic acid, maleic acid and further acids which are commonly employed for forming pharmaceutically acceptable acid addition salts. The base addition salts include salts of the compounds of the present invention with inorganic bases, such as alkali and earth alkaline metal hydroxide or carbonates, or with organic bases, e.g. amines such as mono-, di- or triethanolamine.

If the compounds of the present invention have asymmetric centres, racemates and optical isomers are included as mixtures or in pure form (enantiomers, diastereomers).

The compounds of the present invention can be obtained in a manner known per se.

According to a particular embodiment, the compunds of formula (Ia), wherein R⁸ together with R⁹ form an oxo group, i.e. in particular compounds of formula (Ib), are obtainable via the Biginelli reaction by irradiation of the appropriate aldehyde (IV), urea or thiourea (V) and the appropriate 1,3-dicarbonyl compound (III) together with polyphosphate ester (PPE), e.g. in a microwave oven (Ranu, B. C., Hajra, A., and Dey, S. S. (2002). A Practical and Green Approach towards Synthesis of Dihydropyrimidinones without Any Solvent or Catalyst. Org. Process Res. Dev. 6, 817-818; Cava, M. P., Lakshmikantham, M. V., and Mitchell, M. J. (1969). The Synthesis of Caseadine Methyl Ether. Org. Chem. 34, 2665-2667; Kappe, C. O., and Falsone, S. F. (1998). Polyphosphate Ester-Mediated Synthesis of Dihydropyrimidines. Improved Conditions for the Biginelli Reaction. Synlett, 718-720; Kappe, C. O., Kumar, D., and Varma, R. S. (1999). Microwave-Assisted High-Speed Parallel Synthesis of 4-Aryl-3,4-dihydropyrimidin-2(1*H*)-ones Using a Solventless Biginelli Condensation Protocol. Synthesis, 1799-1803; Kappe, C. O., Shishkin, O. V., Uray, G, and Verdino, P. (2000). X-Ray Structure, Conformational Analysis, Enantioseparation, and Determination of Absolute Configuration of the Mitotic Kinesin Eg5 Inhibitor Monastrol. Tetrahedron 56, 1859-1862). This synthesis is outlined in the following scheme 1:

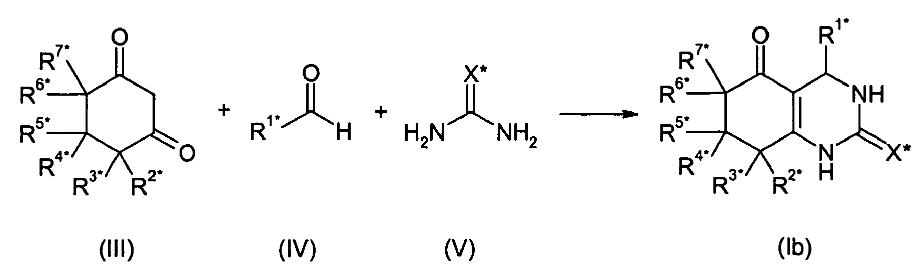

In said scheme as well as in the following schemes, the residues R^{1*}, R^{2*}, R^{3*}, R^{4*}, R^{5*}, R^{6*}, R^{7*}, R^{8*}, X* may have the meanings as indicated for residues R¹, R², R³, R⁴, R⁵, R⁶, R⁷, R⁸, X. However, said residues may also have other meanings and in particular may denote groups which can be converted into the groups R¹, R², R³, R⁴, R⁵, R⁶, R⁷, R⁸, X by well-known chemical reactions such as protection, deprotection, etc.

The present invention thus relates to a process for preparing a quinazoline derivative of formula (Ib), which process comprises:
(i) reacting a compound of the formula (III'): 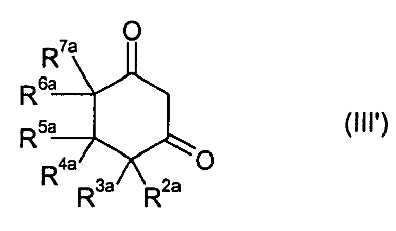 wherein
   R^{2a}, R^{3a}, R^{4a}, R^{5a}, R^{6a}, R^{7a} independently denote hydrogen, halogen, cyano, nitro, hydroxyl, oxo, mercapto, thio, amino, C₁-C₆-alkyl, C₁-C₆-alkoxy, C₁-C₆-alkylthio, C₁-C₆₋alkylamino, di-(C₁-C₆-alkyl)amino, C₁-C₆-haloalkyl, C₁-C₆-haloalkoxy, carboxyl, carbamoyl, C₁-C₆-alkylcarbonyl, C₁-C₆-alkoxycarbonyl, C₁-C₆-alkylcarbonyloxy, C₁-C₆₋alkylaminocarbonyl, di-(C₁-C₆)alkylamincarbonyl, C₁-C₆-alkylsulfinyl, C₁-C₆₋alkylsulfonyl, hydroxysulfonyl, aminosulfonyl, C₁-C₆-alkylaminosulfonyl or di-(C₁-C₆₋alkyl)aminosulfonyl, or R^{3a} together with R^{5a} or R^{5a} together with R^{7a} form a bond, or R^{2a} together with R^{3a}, R^{4a} together with R^{5a}, and/or R^{6a} together with R^{7a}, form an oxo or thio group, wherein R^{2a}, R^{3a}, R^{4a}, R^{5a}, R^{6a} and/or R^{7a} may be protected with an appropriate protecting group,
   with a compound of the formula (IV'): 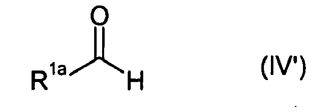 wherein
   R^{1a} denotes phenyl or six-membered heteroaryl, optionally substituted with one to five identical or different substituents selected from the group consisting of halogen, cyano, nitro, hydroxyl, mercapto; C₁-C₆-alkyl, C₁-C₆-alkoxy, C₁-C₆-alkylthio, C₁-C₆-haloalkyl, C₁-C₆-haloalkoxy, carboxyl C₁-C₆-alkylcarbonyl, C₁-C₆-alkoxycarbonyl and C₁-C₆₋alkylcarbonyloxy, wherein R^{1a} may be protected with an appropriate protecting group,
   and with a compound of the formula (V): 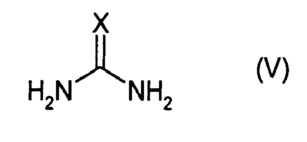 wherein
   X denotes S or O,
   in the presence of polyphosphate ester;
(ii) optionally converting in a manner known per se, e.g. removing protecting groups, the resulting compound of the formula (Ib'): 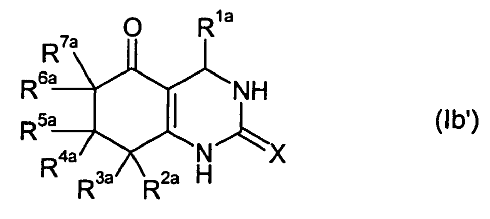 wherein R^{1a}, R^{2a}, R^{3a}, R^{4a}, R^{5a}, R^{6a}, R^{7a}, and X are defined as above,
   into the desired quinazoline derivative of the formula (Ib); and
(iii) recovering the desired quinazoline derivative.

Further, the compound of the formula (Ib) can be converted to the corresponding alcohol of the formula (Ic) by reduction, e.g. by selective Luche-reduction, of the 5-carbonyl function (Gemal, A. L., and Luche, J.-L. (1981). Lanthanoids in Organic Synthesis. 6. The Reduction of α-Enones by Sodium Borohydride in the Presence of Lanthanoid Chlorides: Synthetic and Mechanistic Aspects. J. Am. Chem. Soc. *103*, 5454-5459). This conversion is outlined in the following scheme 2:

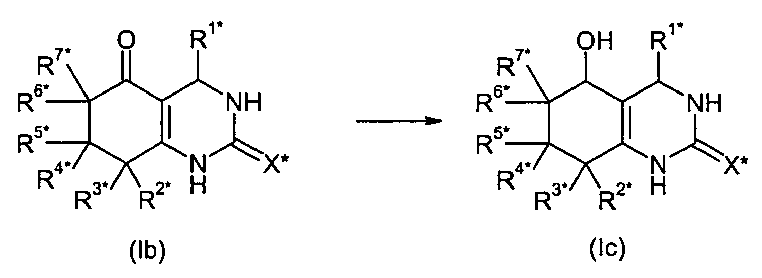

The present invention thus relates to a process for preparing a quinazoline derivative of formula (Ic), which process comprises:
(i) reducing a compound of the formula (Ib'): 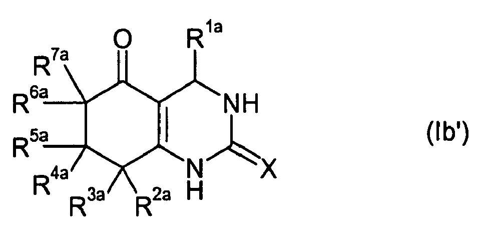 wherein R^{1a}, R^{2a}, R^{3a}, R^{4a}, R^{5a}, R^{6a}, R^{7a}, and X are defined as above,
   e.g. with sodium borohydride in a solvent, such as methanol;
(ii) optionally converting in a manner known per se, e.g. removing protecting groups, the resulting compound of the formula (Ic'): 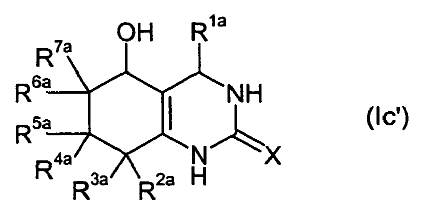 wherein R^{1a}, R^{2a}, R^{3a}, R^{4a}, R^{5a}, R^{6a}, R^{7a}, and X are defined as above,
   into the desired quinazoline derivative of the formula (Ic); and
(iii) recovering the desired quinazoline derivative.

Further, the compounds of formula (Ia), wherein R³ together with R⁵ and R⁷ together with R⁹ form a bond, i.e. in particular the compounds of formula (Id), are obtainable as outlined in the following scheme 3:

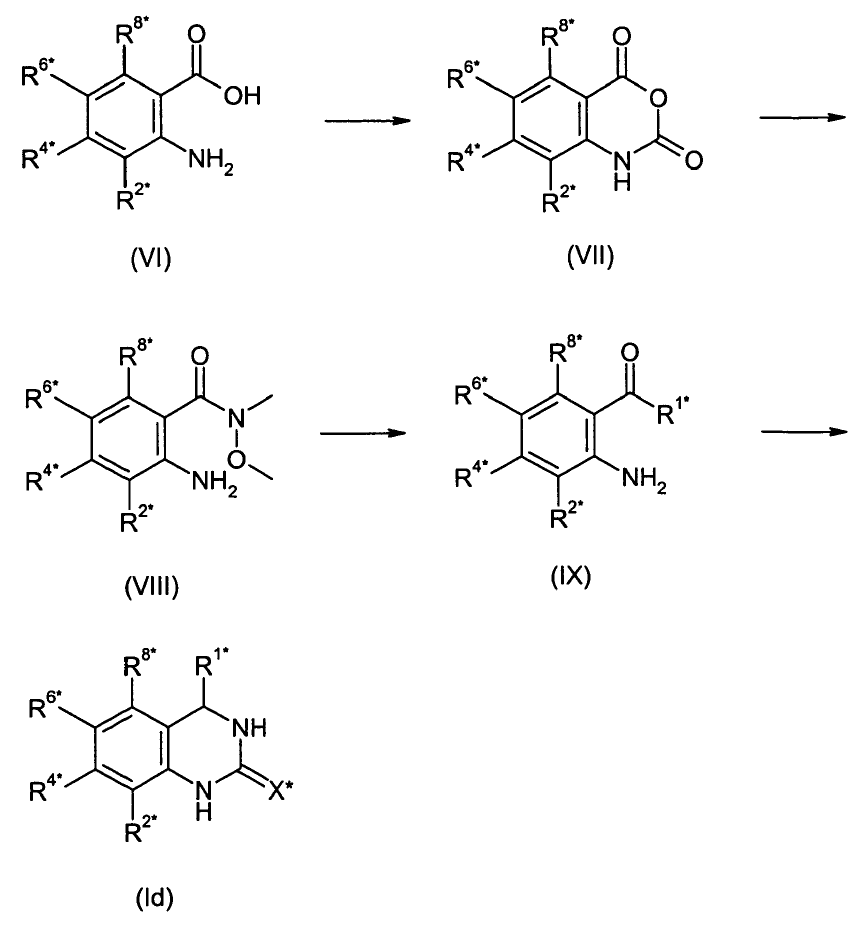

The present invention thus relates to a process for preparing a quinazoline derivative of formula (Id), which process comprises:
(i) reacting a compound of the formula (VI'): 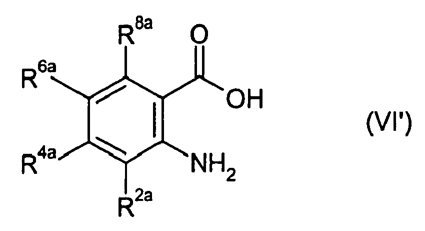 wherein R^{2a}, R^{4a}, R^{6a}, R^{8a} independently denote hydrogen, halogen, cyano, nitro, hydroxyl, mercapto, amino, C₁-C₆-alkyl, C₁-C₆-alkoxy, C₁-C₆-alkylthio, C₁-C₆₋alkylamino, di-(C₁-C₆-alkyl)amino, C₁-C₆-haloalkyl, C₁-C₆-haloalkoxy, carboxyl, carbamoyl, C₁-C₆-alkylcarbonyl, C₁-C₆-alkoxycarbonyl, C₁-C₆-alkylcarbonyloxy, C₁-C₆₋alkylaminocarbonyl, di-(C₁-C₆-alkyl)amincarbonyl, C₁-C₆-alkylsulfinyl, C₁-C₆₋alkylsulfonyl, hydroxysulfonyl, aminosulfonyl, C₁-C₆-alkylaminosulfonyl or di-(C₁-C₆₋alkyl)aminosulfonyl, wherein R^{2a}, R^{4a}, R^{6a} and/or R^{8a} may be protected with an appropriate protecting group,
   with trichloromethyl chloroformate (ClCO₂CCl₃) in a solvent, such as 1,4-dioxane;
(ii) reacting the resulting compound of the formula (VII'): 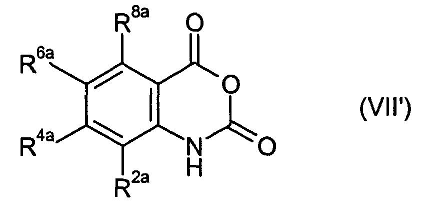 wherein R^{2a}, R^{4a}, R^{6a}, R^{8a} are as defined above,
   with N,O-dimethylhydroxylamine (NH(OCH₃)CH₃) in a solvent, such as ethanol;
(iii) reacting the resulting compound of the formula (VIII'): 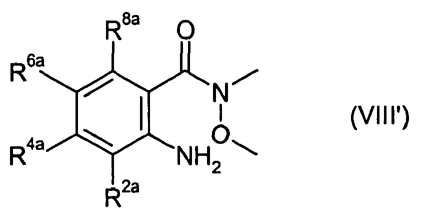 wherein R^{2a}, R^{4a}, R^{6a}, R^{8a} are as defined above,
   with a compound of formula

   R^{1a}-Br

   wherein R^{1a} denotes phenyl or six-membered heteroaryl, optionally substituted with one to five identical or different substituents selected from the group consisting of halogen, cyano, nitro, hydroxyl, mercapto; C₁-C₆-alkyl, C₁-C₆-alkoxy, C₁-C₆-alkylthio, C₁-C₆-haloalkyl, C₁-C₆-haloalkoxy, carboxyl C₁-C₆-alkylcarbonyl, C₁-C₆-alkoxycarbonyl and C₁-C₆-alkylcarbonyloxy, wherein R^{1a} may be protected with an appropriate protecting group,
   in the presence of a base, such as n-butyl lithium, in a solvent, such as tetrahydrofurane;
(iv) reacting the resulting compound of the formula (IX'): 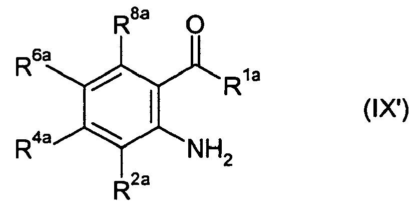 wherein R^{1a}, R^{2a}, R^{4a}, R^{6a}, R^{8a} are as defined above,
   with a reducing agent, such as NaBH₄, and subsequently with a thiocyanate, such NH₄SCN, or with a cyanate, such as NH₄OCN, in the presence of an acid, such as HCl;
(v) optionally converting in a manner known per se the resulting compound of the formula (Id'): 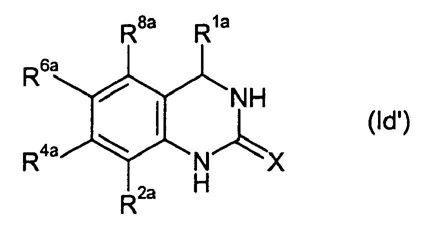 wherein X, R^{1a}, R^{2a}, R^{4a}, R^{6a}, R^{8a} are as defined above,
   into the desired quinazoline derivative of the formula (Id); and
(vi) recovering the desired quinazoline derivative.

Compounds of the present invention are useful as modulators, in particular inhibitors, of the mitotic kinesin Eg5. The term "mitotic kinesin Eg5" includes, but is not limited to, the human protein (also called KSP), the *Xenopus laevis* homologue, further homologues from other species as well as allelic variants and functionally related proteins. The present invention thus also relates to said compounds for use as Eg5 modulators, especially inhibitors. Accordingly, compounds of the present invention can find use in a variety of applications, which in particular include therapeutic applications. The present invention thus relates to compounds for use in therapy.

According to a particular embodiment, compounds of the present invention are used to modulate mitotic spindle formation, thus causing prolonged cell cycle arrest in mitosis. The term "modulate" means altering mitotic spindle formation, including increasing and decreasing spindle formation. The term "mitotic spindle formation" means organisation of microtubules into bipolar structures by mitotic kinesins. Thus, compounds of the present invention are useful as antimitotics, i.e. as drugs that prevent or interfere with mitosis. In particular, said compounds are useful as M phase-specific inhibitors, i.e. they can be used to inhibit the M phase-progression.

In particular, compounds of the present invention are useful for treating cellular proliferation diseases. These include, but are not limited to, cancer, autoimmune diseases, arthritis, graft rejection, inflammatory bowel disease, and proliferation induced after medical procedures, including, but not limited to, surgery and angioplasty.

Compounds of the present invention are particularly useful for the treatment of cancer including solid tumors such as carcinomas.

Compounds according to the invention can either be administered to a subject in of such treatment as individual therapeutic active compounds or as mixtures with other therapeutic active compounds: they can be administered as such, but in general they are administered in the form of pharmaceutical compositions, i.e. as mixtures of the active compounds with pharmaceutically acceptable excipients, in particular vehicles or diluents and/or additives. The compounds or compositions can be administered enterally, e.g. orally or rectally, or parenterally, e.g. subcutaneously, intravenously or intramuscularly.

The nature of the pharmaceutical composition and of the pharmaceutical carrier or diluent depends on the desired manner of administration. Oral compositions can be present, for example, as tablets or capsules and can contain customary excipients, such as binding agents (e.g. syrup, acacia, gelatin, sorbitol, tragacanth or polyvinylpyrrolidone), fillers (e.g. lactose, sugar, maize starch, calcium phosphate, sorbitol or glycine), lubricants (e.g. magnesium stearate, talc, polyethylene glycol or silica), disintegrating agents (e.g. starch) or wetting agents (e.g. sodium laurylsulphate). Oral liquid preparations can be present in the form of aqueous or oily suspensions, solutions, emulsions, syrups, elixirs or sprays etc. or can be present as dry powders for reconstitution with water or another suitable carrier. Liquid preparations of this type can contain customary additives, for example suspending agents, flavourings, diluents or emulsifiers. For parenteral administration, solutions or suspensions with customary pharmaceutical carriers can be employed.

The following examples illustrate the present invention without limiting it.

### Experimental procedures:

### General methods for the chemical synthesis of the compounds.

All reactions were performed with commercially available reagents and they were used without further purification. Solvents were dried by standard methods and stored over molecular sieves. All reactions were monitored by thin-layer chromatography (TLC) carried out on Merck silica gel 60 F₂₅₄ aluminium sheets and viewed with UV light. Flash-chromatography was performed on Merck silica gel 60. The solvent mixtures for flash-chromatography are indicated in the supplementary material as well as the analytical data for all synthesized compounds. Melting points were determined in open capillaries using a Büchi Melting Point B-540 apparatus and are uncorrected. ¹H and ¹³C NMR spectra were recorded on Varian VXR-200, Varian VXR-300 and Bruker DRX-600 NMR spectrometers at room temperature. The solvents stated were used as internal standards. High-resolution (HR) mass spectra were obtained with a 7 T APEX II mass spectrometer.

### General procedure A: Synthesis of 4-(hetero)aryl-5-oxo-3,4,5,6,7,8-hexahydroquinazoline-2(1H)-thiones or 4-(hetero)aryl-5-oxo-3,4,5,6,7,8-hexahydroquinazoline-2(1H)-ones (compounds of the formula (Ib)).

This synthesis was performed as outlined in the following scheme 4:

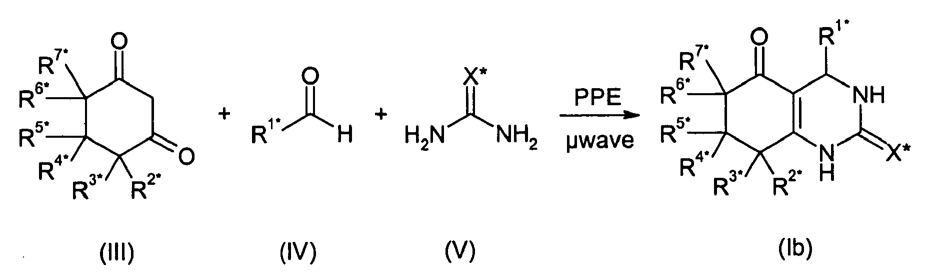

Briefly, phosphorus pentoxide (150 g) was added to a solution of anhydrous diethyl ether (300 ml) and alcohol-free chloroform (150 ml). The reaction mixture was refluxed under argon atmosphere for 4 days. Afterwards the solution was decanted from a small amount of residue and the clear solution was concentrated to a colourless syrup under reduced pressure. Residual traces of solvent were removed by heating the syrup for 72 hours at 40 °C in vacuum, which yielded the desired polyphosphate ester (PPE).

The appropriate β-diketone of formula (III) (6.6 mmol), aldehyde of formula (IV) (6.0 mmol), urea or thiourea (18.0 mmol) and PPE (900 mg) were placed in a 50 ml glass beaker and mixed well. Then the glass beaker was placed in a crystallization dish (11.5 cm diameter) filled with neutral alumina (250 g). This set-up was irradiated on the turntable of a domestic microwave oven (Proline SM20S, 2450 MHz, 800 W) 15 times at full power (800 W) for 5-7 s each with 1-2 min cooling periods in between each irradiation cycle. Afterwards ethanol (10 ml) and water (5 ml) were added to the reaction mixture and the solid was dissolved in an ultrasonic bath. The resulting solution was subsequently poured into 200 ml of stirred ice cold water and the precipitated crude product was filtered off. After drying the crude product in vacuum it was purified by flash-chromatography.

### General procedure B: Synthesis of 4-(hetero)aryl-5-hydroxy-3,4,5,6,7,8-hexahydroquinazoline-2(1H)-thiones or 4-(hetero)aryl-5-hydroxy-3,4,5,6,7,8-hexahydroquinazoline-2(1H)-ones (compounds of the formula (Ic)).

This synthesis was performed as outlined in the following scheme 5:

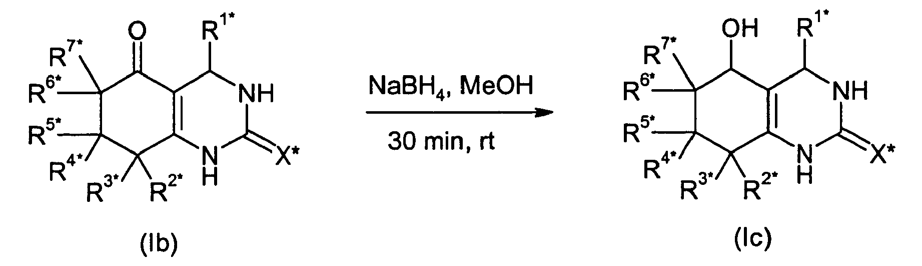

Briefly, to a suspension of the appropriate 4-(hetero)aryl-5-oxo-3,4,5,6,7,8-hexahydroquinazoline-2(1*H*)-thione or 4-(hetero)aryl-5-oxo-3,4,5,6,7,8-hexahydroquinazoline-2(1*H*)-one (compounds of the formula (lb); 0.36 mmol) and CeCl₃·7H₂O (0.36 mmol) in dry methanol (3.6 ml) under argon atmosphere was added sodium borohydride (0.36 mmol) in one portion at room temperature. The reaction mixture was then stirred at this temperature for 30 min. Afterwards water (3 ml) was added and the resulting suspension was evaporated to dryness. Flash-chromatography of the crude product (CHCl₃/CH₃OH 20:1) yielded the product.

### General procedure C: Synthesis of 3,4-Dihydro-4-(hetero)aryl-quinazoline-2(1H)-thiones.

### a) Isatoic anhydrides of the formula (VII).

Isatoic anhydrides of formula (VII) were synthesised according to M. H. Norman, G. C. Rigdon, W. R. Hall, F. Navas, *J. Med. Chem*. 1996, *39*, 1172-1188, and were used in the next step without isolation. This synthesis is outlined in the following scheme 6:

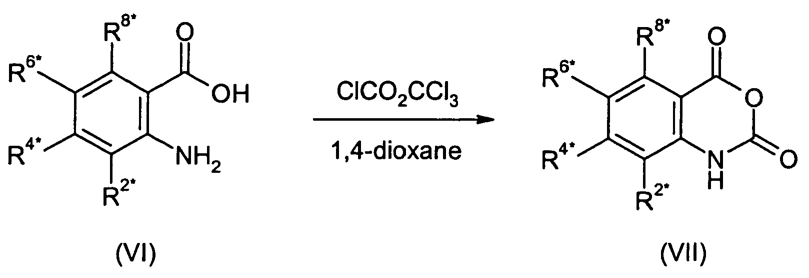

Briefly, the appropriate 2-amino-benzoic acid (compound of formula (VI); 6.25 mmol), anhydrous 1,4-dioxane (20 mL), and trichloromethyl chloroformate (5.0 g, 25.2 mmol, 4.0 eq) were added to a 100-mL round-bottomed flask. The reaction mixture was heated at reflux for 11 h. The reaction mixture was allowed to cool and stir at room temperature overnight. The solvent was removed with a rotary evaporator to give the crude product. This material was used without further purification.

### b) N-Methoxy-N-methylamides of the formula (VIII).

N-Methoxy-N-Methylamides of the formula (VIII) were prepared from the corresponding isatoic anhydrides according to S. V. Frye, M. C. Johnson, N. L. Valvano, *J. Org. Chem.* 1991, 56, 3750-3752. This synthesis is outlined in the following scheme 7:

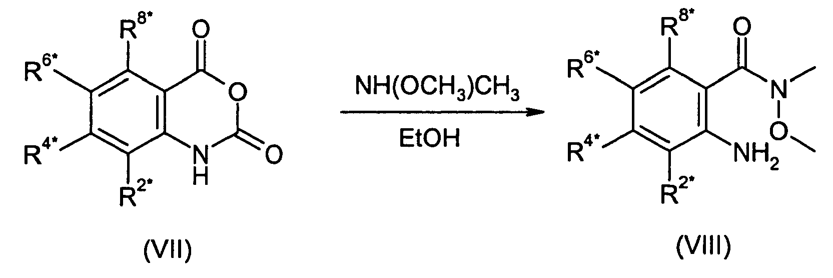

Briefly, to a solution of N,O-dimethylhydroxylamine hydrochloride (5.12 g, 0.053 mol) in 90% aqueous ethanol (20 mL) was added triethylamine (5.3 g, 0.053 mol), and, after 10 min of stirring at 25 °C, the appropriate isatoic anhydride (compound of formula (VII); 0.035 mol) was added in portions. The reaction was then heated at reflux for 1.5 h and poured onto an equal volume of ice and saturated sodium bicarbonate. The ethanol was then removed by rotary evaporation, the resulting aqueous mixture was extracted with ethyl acetate (3 X 20 mL), dried over magnesium sulfate and activated charcoal, and concentrated. If an oil was obtained, this was chromatographed on silica gel (e.g. 1:I diethyl ether/hexanes, then acetone) to yield the product.

### c) 2-Aminobenzophenones of the formula (IX).

2-Aminobenzophenones of the formula (IX) were prepared from the corresponding N-methoxy-N-methylamides according to S. V. Frye, M. C. Johnson, N. L. Valvano, J. *Org. Chem.* 1991, 56, 3750-3752. This synthesis is outlined in the following scheme 8:

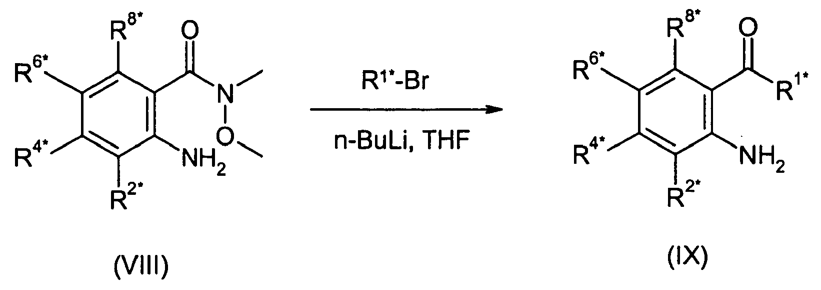

Briefly, to a mixture of the appropriate N-methoxy-N-methylamide (compound of formula (VIII), 11.1 mmol) and the appropriate (hetero)arylbromide (compound of the formula R^{1a}-Br; 11.1 mmol) in anhydrous tetrahydrofuran (65 mL) at -78 °C under nitrogen was added, with vigorous stirring, n-BuLi in hexanes (13.8 mL, 1.6 M, 22.2 mmol) at 0.6 mL/min. After 20 min, aqueous hydrochloric acid was added (1 N, 20 mL), the mixture was extracted with ethyl acetate (150 mL), and the ethyl acetate was washed with water and brine, dried over magnesium sulfate, and concentrated. If an oil was obtained, this was chromatographed on silica gel (hexane / ethyl acetate, 1:1) to afford the product.

### d) 3,4-Dihydro-4-(hetero)aryl-quinazoline-2(1H)-thiones of the formula (Id).

3,4-Dihydro-4-(hetero)aryl-quinazoline-2(1*H*)-thiones of the formula (Id) were synthesised according to D. Xu, P. G. Kucerovy, K. Prasad, O. Repic, *Tetrahedron Asymmetry* 1996, 7, 747-754. This synthesis is outlined in the following scheme 9:

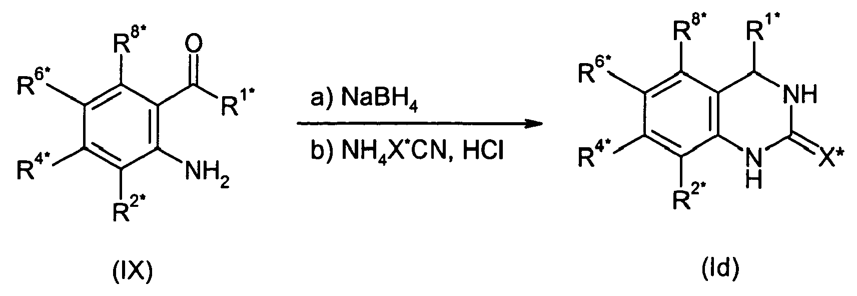

Briefly, a solution of the appropriate 2-aminobenzophenone (compound of formula (IX), 1.0 mmol) in 10 ml of 95% ethanol was heated to 65 °C under a nitrogen atmosphere. To the mixture was added portionwise over 5 min 38 mg (1.0 mmol) of NaBH₄, and the mixture was stirred at 65-70 °C for 1.5 h. The resulting off-white suspension was diluted with 5 ml of water and followed by the drop wise addition of a solution of 92 mg (1.2 mol) of ammonium thiocyanate (NH₄XCN with X = S) in 1 ml of water. The temperature was maintained at 65 °C throughout the addition. The reaction mixture was then treated with 2 ml of concentrated hydrochloric acid in 1 ml of water and stirred at 65 -70 °C for 2 h. The mixture was cooled to 45 °C and filtered. The solid was washed with 2 ml of warm water and dried to afford the product.

### Reference example 1: 2-Amino-N-methoxy-N-methylbenzamide.

### Method: General procedure C b)

### Starting compound: Isatoic anhydride

Physical and spectroscopic data for this compound are identical with those already published in S. V. Frye, M. C. Johnson, N. L. Valvano, *J. Org. Chem.* **1991,** 56, 3750-3752.

### Reference example 2: 2-Amino-4-chloro-N-methoxy-N-methylbenzamide.

### Method: General procedure C b)

### Starting compound: 4-Chloroisatoic anhydride

¹H NMR (300 MHz, CDCl₃): δ (ppm) = 3.30 (3H, s), 3.53 (3H, s), 4.78 (2H, brs), 6.61 (1H, dd, *J*=1.9, 8.2Hz), 6.67 (1H, d, *J*=1.9Hz), 7.30 (1H, d, *J*=8.2Hz);
¹³C NMR (75 MHz,: CDCl₃): δ (ppm) = -34.2, 61.4, 115.3, 116.4, 117.0, 130.9, 137.4, 148.6, 169.5;
ESI-HRMS *m*/*z*: calcd for C₉H₁₁ClN₂O₂Na (MNa⁺) 237.04013, found 237.03956; oil.

### Reference example 3: 2-Amino-N-methoxy-N,3-dimethylbenzamide.

### Method: General procedure C b)

### Starting compound: 3-Methylisatoic anhydride

¹H NMR (300 MHz, CDCl₃): δ (ppm) = 2.1 (3H, s), 3.32 (3H, s), 3.57 (3H, s), 4.60 (2H, brs), 6.60 (1H, t, *J*=7.1Hz), 7.06 (1 H, d, *J*=7.1 Hz), 7.2 (1 H, d, *J*=7.1 Hz);
¹³C NMR (75 MHz,: CDCl₃): δ (ppm) = -17.8, 34.9, 61.3, 116.6, 117.1, 123.4, 127.2, 132.6, 145.1, 170.8;
ESI-HRMS *m*/*z*: calcd for C₁₀H₁₄N₂O₂Na (MNa⁺) 217.09475, found 217.09468; oil.

### Reference example 4: 2-Amino-5-chloro-N-methoxy-N-methylbenzamide.

### Method: General procedure C b)

### Starting compound: 5-Chloroisatoic anhydride

¹H NMR (200 MHz, CDCl₃): δ (ppm) = 3.33 (3H, s), 3.57 (3H, s), 4.56 (2H, brs), 6.63 (1 H, d, *J*= 8.6 Hz), 7.12 (1H, dd, *J*=2.5, 8.6Hz), 7.35 (1H, d, *J*=2.5Hz);
¹³C NMR (50 MHz,: CDCl₃): δ (ppm) = -35.9, 63.3, 119.9, 120.2, 123.4, 130.7, 133.3, 147.4, 170.6;
ESI-HRMS *m*/*z*: calcd for C₉H₁₂ClN₂O₂ (MH⁺) 215.05818, found 215.05835
m.p.= 69-72°C.

### Reference example 5: 2-Amino-N,3-dimethoxy-N-methylbenzamide.

### Method: General procedure C b)

### Starting compound: 3-Methoxyisatoic anhydride

¹H NMR (300 MHz, CDCl₃): δ (ppm) = 3.30 (3H, s), 3.57 (3H, s), 3.82 (3H, s), 4.77 (2H, brs), 6.61 (1 H, t, *J*=7.9Hz), 6.77 (1 H, d, *J*=7.7Hz), 6.96 (1H, d, *J*=7.9Hz);
¹³C NMR (75 MHz,: CDCl₃): δ (ppm) = -34.5, 55.7, 61.1, 111.4, 115.9, 117.0, 120.9, 137.4, 147.5, 170.0;
ESI-HRMS *m*/*z*: calcd for C₁₀H₁₅N₂O₃ (MH⁺) 211.10772, found 211.10776; oil.

### Reference example 6: 2-Amino-4-fluoro-N-methoxy-N-methylbenzamide.

### Method: General procedure C b)

### Starting compound: 4-Fluoroisatoic anhydride

¹H NMR (300 MHz, CDCl₃): δ (ppm) = 3.32 (3H, s), 3.55 (3H, s), 4.71 (2H, brs), 6.32-6.38 (2H, m), 7.37-7.41 (1H, m);
¹³C NMR (75 MHz,: CDCl₃): δ (ppm) = -34.2, 61.2, 102.9(d, *J*=24,6Hz), 104.0(d, *J*=22.3Hz), 112.8(d, *J*=2.3Hz), 131.8(d, *J*=10.9Hz), 149.7(d, *J*=11.5Hz), 164.7(d, *J*=148.5Hz), 169.4;
ESI-HRMS *m*/*z*: calcd for C₉H₁₁FN₂O₂Na (MNa⁺) 221.06968, found 221.06982; oil.

### Reference example 7: 2-Amino-5-bromo-N-methoxy-N-methylbenzamide.

### Method: General procedure C b)

### Starting compound: 5-Bromoisatoic anhydride

¹H NMR (200 MHz, CDCl₃): δ (ppm) = 3.33 (3H, s), 3.58 (3H, s), 4.68 (2H, brs), 6.61 (1 H, d, *J*=8.8Hz), 7.25 (1H, dd, *J*=2.6, 8.8Hz), 7.49 (1H, d, *J*= 2.6Hz);
¹³C NMR (50 MHz,: CDCl₃): δ (ppm) = 34.0, 61.4, 108.3, 118.7, 118.3, 131.7, 134.2, 146.0, 168.6
ESI-HRMS *m*/*z*: calcd for C₉H₁₁BrN₂NaO₂ (MNa⁺) 280.98961, found 280.98980;
m.p.= 72-74°C.

### Reference example 8: (2-Aminophenyl)(3'-tert-butyldimethylsilyloxyphenyl)methanone.

### Method: General procedure C c)

### Starting compound: 2-Amino-N-methoxy-N-methylbenzamide (reference example 1)

### Reactant: (3-Bromophenoxy)(tert-butyl)dimethylsilane

¹H NMR (200 MHz, CDCl₃): δ (ppm) = 0.22 (6H, s), 0.99 (9H, s), 6.11 (2H, brs), 6.57-6.65 (1 H, m), 6.74 (1H, d, *J=* 8.4), 7.01 (1 H, m), 7.10 (1 H, m), 7.19-7.36 (3H, m), 7.47 (1 H, dd, *J*=1.5, 8.1 Hz)
¹³C NMR (50 MHz,: CDCl₃): δ (ppm) = -4.2, 18.4, 25.8, 115.6, 117.1, 118.3, 120.7, 122.3, 123.0, 129.3, 134.4, 134.8, 141.6, 151.1, 155.5, 198.9;
ESI-HRMS *m*/*z*: calcd for C₁₉H₂₆NO₂Si (MH⁺) 328.17273, found 328.17280; oil.

### Reference example 9: (2-Amino-4-chlorophenyl)(3'-tert-butyl-dimethylsilyloxyphenyl)methanone.

### Method: General procedure C c)

### Starting compound: 2-Amino-4-chloro-N-methoxy-N-methylbenzamide (reference example 2)

### Reactant: (3-Bromophenoxy)(tert-butyl)dimethylsilane

¹H NMR (300 MHz, CDCl₃): δ (ppm) = 0.21 (6H, s), 0.99 (9H, s), 6.20 (2H, brs), 6.56 (1 H, dd, *J*=1.9, 8.7Hz), 6.73 (1H, d, *J*=1.9Hz), 6.99-7.07 (2H, m), 7.18 (1H, d, *J*=7.7Hz), 7.26-7.34 (1H, m), 7.40 (1H, d, *J*=8.8Hz);
¹³C NMR (75 MHz,: CDCl₃): δ (ppm) = --4.1, 18.5, 25.9, 116.2, 116.5, 116.8, 120.7, 122.3, 123.3, 129.6, 136.2, 140.6, 141.4, 152.0, 155.7, 198.2
ESI-HRMS *m*/*z*: calcd for C₁₉H₂₅ClNO₂Si (MH⁺) 362.13376, found 362.13355; oil.

### Reference example 10: (2-Amino-3-methylphenyl)(3'-tert-butyl-dimethylsilyloxyphenyl)methanone.

### Method: General procedure C c)

### Starting compound: 2-Amino-N-methoxy-N,3-dimethylbenzamide (reference example 3)

### Reactant: (3-Bromophenoxy)(tert-butyl)dimethylsilane

¹H NMR (300 MHz, CDCl₃): δ (ppm) = 0.22 (6H, s), 1.00 (9H, s), 2.22 (3H, s), 6.21 (2H, brs), 6.55 (1 H, t, *J*=7.7Hz), 6.99-7.02 (1 H, m), 7.11 (1H, m), 7.21-7.33 (3H, m), 7.38 (1H, d, *J*=7.4Hz);
¹³C NMR (75 MHz,: CDCl₃): δ (ppm) = -4.1, 18.5, 17.6, 26.0, 115.1, 117.8, 120.9, 122.5, 123.0, 123.5, 129.4, 133.1, 135.4, 142.1, 149.7, 155.6, 199.5;
ESI-HRMS *m*/*z*: calcd for C₂₀H₂₇NO₂SiNa (MNa⁺) 364.17033, found 364.17020; oil.

### Reference example 11: (2-Amino-5-chlorophenyl)(3'-tert-butyldimethylsilyloxyphenyl)methanone.

### Method: General procedure C c)

### Starting compound: 2-Amino-5-chloro-N-methoxy-N-methylbenzamide (reference example 4)

### Reactant: (3-Bromophenoxy)(tert-butyl)dimethylsilane

¹H NMR (300 MHz, CDCl₃): δ (ppm) = 0.23 (6H, s), 0.99 (9H, s), 6.04 (2H, brs), 6.67 (1 H, d, *J*=8.8Hz), 7.01-7.04 (1 H, m), 7.07-7.09 (1H, m), 7.19-7.26 (2H, m), 7.33 (1H, t, J=7.7Hz), 7.44 (1 H, d, J=2.5Hz);
¹³C NMR (75 MHz,: CDCl₃): δ (ppm) = -4.1, 18.5, 25.9, 118.7, 119.0, 120.2, 120.8, 122.4, 123.7, 129.7, 133.5, 134.5, 140.9, 149.6, 155.8, 197.9;
ESI-HRMS *m*/*z*: calcd for C₁₉H₂₅ClNO₂Si (MH⁺) 362.13376, found 362.13381; oil.

### Reference example 12: (2-Amino-3-methoxyphenyl)(3'-tert-butyldimethyloxyphenyl)methanone.

### Method: General procedure C c)

### Starting compound: 2-Amino-N,3-dimethoxy-N-methylbenzamide (reference example 5)

### Reactant: (3-Bromophenoxy)(tert-butyl)dimethylsilane

¹H NMR (200 MHz, CDCl₃): δ (ppm) = 0.23 (6H, s), 1.00 (9H, s), 3.90 (3H, s), 6.37 (2H, brs), 6.50-6.62 (2H, m), 6.82-6.90 (1H, t, *J*=8.1Hz), 6.98-7.02 (1H, m), 7.09-7.12 (1 H, m), 7.20-7.41 (2H, m);
¹³C NMR (50 MHz,: CDCl₃): δ (ppm) = -4.3, 18.3, 25.8, 55.9, 113.8, 114.0, 117.5, 120.6, 122.2, 122.7, 122.8, 126.1, 129.1, 141.8, 147.4, 155.4, 198.7;
ESI-HRMS *m*/*z*: calcd for C₂₀H₂₇NO₃Si (MH⁺) 358.18330, found 358.18324; oil.

### Reference example 13: (2-Amino-4-fluorophenyl)(3'-tert-butyldimethylsilyloxyphenyl)methanone.

### Method: General procedure C c)

### Starting compound: 2-Amino-4-fluoro-N-methoxy-N-methylbenzamide (reference example 6)

### Reactant: (3-Bromophenoxy)(tert-butyl)dimethylsilane

¹H NMR (300 MHz, CDCl₃): δ (ppm) = 0.21 (6H, s), 0.98 (9H, s), 6.26-6.40 (3H, m), 6.36 (1 H, d, *J*=2.5, 10.9Hz), 6.98-7.05 (2H, m), 7.17 (1 H, d, *J*=7.4Hz), 7.26-7.33 (1 H, m), 7.47 (1 H, dd, *J*=6.6, 8.8Hz);
¹³C NMR (50 MHz,: CDCl₃): δ (ppm) = -4.27, 18.33, 25.8, 102.5(d, *J*=24.1Hz), 103.8(d, *J*=22.9Hz), 115.1, 120.5, 122.1, 123.0, 129.4, 137.7(d, *J*=11.5Hz), 141.6, 153.5(d, *J*=13.2 Hz), 155.6, 166.1(d, *J*=253.7), 197.8
ESI-HRMS *m*/*z*: calcd for C₁₉H₂₅F NO₂Si (MH⁺) 346.16331 found 346.16336; oil.

### Reference example 14: (2-Amino-5-bromophenyl)(3'-tert-butyldimethylsilyloxyphenyl)methanone.

### Method: General procedure C c)

### Starting compound: 2-Amino-5-bromo-N-methoxy-N-methylbenzamide (reference example 7)

### Reactant: (3-Bromophenoxy)(tert-butyl)dimethylsilane

¹H NMR (300 MHz, CDCl₃): δ (ppm) = 0.24 (6H, s), 0.99 (9H, s), 6.10 (2H, brs), 6.63 (1H, d, *J*=8.5Hz), 7.01-7.07 (2H, m), 7.19-7.36 (3H, m), 7.57 (1H, brd);
¹³C NMR (75 MHz,: CDCl₃): δ (ppm) = -4.3, 18.2, 25.7, 106.5, 118.8, 119.3, 120.5, 122.1, 123.4, 129.4, 136.2, 136.8, 140.5, 149.7, 155.5, 197.5;
ESI-HRMS *m*/*z*: calcd for C₁₉H₂₅BrNO₂Si (MH⁺) 406.08324, found 406.08343; oil.

### Example 1: 4-(3-Hydroxyphenyl)-5-oxo-3,4,5,6,7,8-hexahydroquinazoline-2(1H)-thione.

### Method: General procedure A

### Starting compound: Cyclohexan-1,3-dion

### Reactants: 3-Hydroxybenzaldehyde / thiourea

Yellow solid; m.p. 283 °C (decomp.); flash-chromatography: CH₂Cl₂/CH₃OH 20:1; *R*_{*f*} = 0.34 (CH₂Cl₂/CH₃OH 15:1);
¹H NMR (200 MHz, [D6]DMSO): δ (ppm) = 1.71-2.02 (m, 2H, CH₂), 2.21-2.25 (m, 2H, CH₂), 2.41-2.57 (m, 2H, CH₂), 5.08 (d, ³*J* = 3.2 Hz, 1H, CH), 6.59-6.65 (m, 3H, 3x CH_{arom.}), 7.04-7.11 (m, 1 H, CH_{arom.}), 9.35 (s, 1H, OH), 9.58 (br, 1H, NH), 10.52 (br, 1H, NH);
¹³C NMR (50 MHz, [D6]DMSO): δ (ppm) = 20.5 (CH₂), 25.3 (CH₂), 36.3 (CH₂), 51.6 (CH), 109.1 (*C*-COCH₂), 113.2 (CH_{arom.}), 114.3 (CH_{arom.}), 116.9 (CH_{arom.}), 129.3 (CH_{arom.}), 144.6 (C_{arom.}), 150.5 (*C*-CH₂), 157.3 (*C*-OH), 174.4 (C=S), 193.8 (C=O); HRESIMS for C₁₄H₁₄N₂O₂S [M+H]⁺: calcd. 275.0849, found 275.0851.

### Example 2: 7,7-Dimethyl-4-(3-hydroxyphenyl)-5-oxo-3,4,5,6,7,8-hexahydroquinazoline-2(1H)-thione.

### Method: General procedure A

### Starting compound: 5,5-Dimethylcyclohexan-1,3-dion

### Reactants: 3-Hydroxybenzaldehyde / thiourea

Yellow solid; m.p. 220 °C (decomp.); flash-chromatography: CH₂Cl₂/CH₃OH 20:1; *R*_{*f*} = 0.31 (CH₂Cl₂/CH₃OH 15:1);
¹H NMR (300 MHz, [D6]DMSO): δ (ppm) = 0.92 (s, 3H, CH₃), 1.05 (s, 3H, CH₃), 2.06-2.53 (m, 4H, 2x CH₂), 5.10 (d, ³*J* = 3.3 Hz, 1 H, CH), 6.64-6.68 (m, 3H, 3x CH_{arom.}), 7.10-7.15 (m, 1 H, CH_{arom.}), 9.46 (s, 1H, OH), 9.66 (br, 1H, NH), 10.57 (br, 1H, NH);
¹³C NMR (75 MHz, [D6]DMSO): δ (ppm) = 32.2 (CH₃), 34.3 (CH₃), 37.7 (CH₂), 44.0 (*C*(CH₃)₂), 55.3 (CH₂), 57.5 (CH), 113.6 (*C*-COCH₂), 118.8 (CH_{arom.}), 119.9 (CH_{arom.}), 122.4 (CH_{arom.}), 134.9 (CH_{arom.}), 150.2 (C_{arom.}), 154.0 (*C*-CH₂), 162.9 (*C*-OH), 180.0 (C=S), 199.1 (C=O);
HRESIMS for C₁₆H₁₈N₂O₂S [M+H]⁺: calcd. 303.1162, found 303.1164.

### Example 3: 4-(3-Hydroxyphenyl)-5-hydroxy-3,4,5,6,7,8-hexahydroquinazoline-2(1H)-thione.

### Method: General procedure B

### Starting compound: 4-(3-Hydroxyphenyl)-5-oxo-3,4,5,6,7,8-hexahydroquinazoline-2(1H)-thione (example 1)

Yellow solid; m.p. 130 °C; *R*_{*f*} = 0.34 (CH₂Cl₂/CH₃OH 10:1); Diastereomeric mixture: ratio 3:1;
Major diastereomer (aromatic ring and hydroxy group on different sides):
¹H NMR (600 MHz, [D6]DMSO): δ (ppm) = 1.40-1.49 (m, 2H, CH₂), 1.52-1.60 (m, 2H, CH₂), 1.96-2.04 (m, 2H, CH₂), 3.63-3.66 (m, 1H, CHOH), 4.73 (d, ³*J* = 6.5 Hz, 1 H, CHO*H*), 4.93 (s, 1H, CH), 6.63-6.69 (m, 3H, 3x CH_{arom.}), 7.10-7.14 (m, 1 H, CH_{arom.}), 8.80 (s, 1H, NH), 9.42 (s, 1H, OH), 9.47 (s, 1H, NH);
¹³C NMR (150 MHz, [D6]DMSO): δ (ppm) = 17.7 (CH₂), 25.0 (CH₂), 31.8 (CH₂), 54.7 (CH), 62.1 (CH-OH), 110.0 (C-CH(OH)), 113.6 (CH_{arom.}), 114.4 (CH_{arom.}), 117.4 (CH_{arom.}), 129.3 (CH_{arom.}), 129.7 (*C*-CH₂), 144.7 (C_{arom.}), 157.5 (C-OH), 173.2 (C=S). Minor diastereomer (aromatic ring and hydroxy group at the same side):
¹H NMR (600 MHz, [D6]DMSO): δ (ppm) = 1.65-1.71 (m, 2H, CH₂), 1.73-1.79 (m, 2H, CH₂), 2.11-2.16 (m, 2H, CH₂), 3.70-3.73 (m, 1 H, C*H*OH), 4.57 (d, ³*J* = 3.1 Hz, 1H, CH), 4.93-4.95 (m, 1 H, CHO*H*), 6.63-6.69 (m, 3H, 3x CH_{arom.}), 7.10-7.14 (m, 1H, CH_{arom.}), 8.91 (s, 1H, NH), 9.44 (s, 1H, OH), 9.56 (s, 1H, NH);
¹³C NMR (150 MHz, [D6]DMSO): δ (ppm) = 17.6 (CH₂), 27.0 (CH₂), 31.9 (CH₂), 54.7 (CH), 63.8 (CH-OH), 110.0 (C-CH(OH)), 113.6 (CH_{arom.}), 114.4 (CH_{arom.}), 117.6 (CH_{arom.}), 129.3 (CH_{arom.}), 133.6 (*C*-CH₂), 144.2 (C_{arom.}), 157.6 (C-OH), 173.2 (C=S); HRESIMS for C₁₄H₁₆N₂O₂S [M+H]⁺: calcd. 277.1011, found 277.1012.

### Example 4: 3,4-Dihydro-4-(3'-hydroxyphenyl)quinazoline-2(1H)-thione.

### Method: General procedure C d)

Starting compound: (2-Aminophenyl)(3'-*tert*-butyldimethylsilyloxyphenyl)-methanone (reference example 8)

### Reactant: Ammonium thiocyanate

¹H NMR (200 MHz, [D6]DMSO): δ (ppm) = 5.52 (1H, d, *J*=3.0Hz), 6.64-6.75 (3H, m), 6.97-7.00 (2H, m), 7.03-7.20 (3H, m), 9.19 (1H, brs), 9.50 (1H, s), 10.65 (1 H, brs);
¹³C NMR (50 MHz, [D6]DMSO): δ (ppm) = 56.6, 113.1, 114.2, 114.5, 116.8, 121.2, 123.0, 126.9, 128.1, 129.5, 134.1, 145.7, 157.5, 174.4;
ESI-HRMS *m*/*z*: calcd for C₁₄H₁₃N₂OS (MH⁺) 257.07431, found 257.07419;
m.p.= 217-219°C.

### Example 5: 7-Chloro-3,4-dihydro-4-(3'-hydroxyphenyl)quinazoline-2(1H)-thione. Method: General procedure C d)

### Starting compound: (2-Amino-4-chlorophenyl)(3'-tert-butyl-dimethylsilyloxyphenyl)methanone (reference example 9)

### Reactant: Ammonium thiocyanate

¹H NMR (200 MHz, [D6]DMSO): δ (ppm) = 5.5 (1H, d, *J*=2.6Hz), 6.61-6.70 (3H, m), 6.96-7.14 (4H, m), 9.30 (1H, brs), 9.47 (1H, s), 10.70 (1 H, brs);
¹³C NMR (50 MHz, [D6]DMSO): δ (ppm) = 56.2, 113.1, 113.6, 114.7, 116.8, 120.2, 122.7, 128.8, 129.7, 132.4, 135.5, 145.3, 157.7, 174.6;
ESI-HRMS *m*/*z*: calcd for C₁₄H₁₁ClN₂OSNa (MNa⁺) 313.01724, found 313.01700;
m.p.= 93-95°C.

### Example 6: 3,4-Dihydro-4-(3'-hydroxyphenyl)-8-methylquinazoline-2(1H)-thione. Method: General procedure C d)

### Starting compound: (2-Amino-3-methylphenyl)(3'-tert-butyl-dimethylsilyloxyphenyl)methanone (reference example 10)

### Reactant: Ammonium thiocyanate

¹H NMR (300 MHz, [D6]DMSO): δ (ppm) = 2.31 (3H, s), 5.49 (1H, d, *J*=3.3Hz), 6.66-6.68 (2H, m), 6.74 (1H, d, *J*=7.7Hz), 6.93 (1 H, t, *J*=7.4Hz), 7.03-7.16 (3H, m), 9.33 (1H, brs), 9.49 (1H, s), 9.52(1H, brs);
¹³C NMR (75 MHz, [D6]DMSO): δ (ppm) = 17.8, 57.4, 113.9, 115.2, 117.5, 122.6, 123.4, 123.8, 125.5, 130.2, 130.6, 133.1, 146.1, 158.2, 176.0;
ESI-HRMS *m*/*z*: calcd for C₁₅H₁₄N₂OS (M⁺) 270.08268, found 270.08101;
m.p.= 255-256°C.

### Example 7: 6-Chloro-3,4-dihydro-4-(3'-hydroxyphenyl)quinazoline-2(1H)-thione. Method: General procedure C d)

### Starting compound: (2-Amino-5-chlorophenyl)(3'-tert-butyldimethylsilyloxyphenyl)methanone (reference example 11)

### Reactant: Ammonium thiocyanate

¹H NMR (300 MHz, [D6]DMSO): δ (ppm) = 5.55 (1 H, d, *J*=3.3Hz), 6.68-6.69 (2H, m), 6.75 (1H, d, *J*=7.5Hz), 7.04 (1H, d, *J*=9.0Hz), 7.14-7.19 (1H, m), 7.26-7.29 (2H, m), 9.30 (1H, brs), 9.56 (1H, s), 10.79(1H, brs);
¹³C NMR (75 MHz, [D6]DMSO): δ (ppm) = 56.1, 113.0, 114.7, 115.9, 116.7, 123.3, 126.4, 126.5, 128.1, 129.7, 133.1, 145.2, 157.6, 174.5;
ESI-HRMS *m*/*z*: calcd for C₁₄H₁₂ClN₂OS (MH⁺) 291.03534, found 291.03518;
m.p.= 108-110°C.

### Example 8: 3,4-Dihydro-4-(3'-hydroxyphenyl)-8-methoxyquinazoline-2(1H)-thione. Method: General procedure C d)

### Starting compound: (2-Amino-3-methoxyphenyl)(3'-tert-butyldimethyloxyphenyl)methanone (reference example 12)

### Reactant: Ammonium thiocyanate

¹H NMR (300 MHz, [D6]DMSO): δ (ppm) = 5.52 (1 H, d, *J*=3.03Hz), 6.67-6.78 (4H, m), 6.93-7.02 (2H, m), 7.11-7.16 (1H, m), 8.83 (1 H, brs), 9.38 (1H, brs), 9.51 (1 H, s);
¹³C NMR (50 MHz, [D6]DMSO): δ (ppm) = 56.6, 55.9, 110.1, 113.1, 114.5, 116.8, 118.6, 121.8, 123.0, 123.5, 129.5, 144.7, 145.1, 157.5, 174.2;
ESI-HRMS *m*/*z*: calcd for C₁₅H₁₄N₂O₂SNa (MNa⁺) 309.06682, found 309.06711;
m.p.= 254-256°C.

### Example 9: 7-Fluoro-3,4-dihydro-4-(3'-hydroxyphenyl)quinazoline-2(1H)-thione. Method: General procedure C d)

### Starting compound: (2-Amino-4-fluorophenyl)(3'-tert-butyldimethylsilyloxyphenyl)methanone (reference example 13)

### Reactant: Ammonium thiocyanate

¹H NMR (300 MHz, [D6]DMSO): δ (ppm) = 5.53 (1 H, d, *J*=3.0Hz), 6.66-6.84 (5H, m), 7.12-7.20 (2H, m), 9.34 (1H, brs), 9.51 (1H, s), 10.74 (1 H, brs);
¹³C NMR (75 MHz, [D6]DMSO): δ (ppm) = 56.2, 100.9 (d, *J*=26.3), 109.6 (d, *J*=21.8), 113.1, 114.6, 116.8, 117.1 (d, *J*=2.5Hz),128.9 (d, *J*=9.7), 129.7, 135.6 (d, *J*=11.5Hz), 145.5, 157.6, 161.6 (d, *J*=242.2Hz), 174.5;
ESI-HRMS *m*/*z*: calcd for C₁₄H₁₁FN₂OSK (MK⁺) 313.02077, found 313.02087;
m.p.= 85-87°C.

### Example 10: 6-Bromo-3,4-dihydro-4-(3'-hydroxyphenyl)quinazoline-2(1H)-thione. Method: General procedure C d)

### Starting compound: (2-Amino-5-bromophenyl)(3'-tert-butyldimethylsilyloxyphenyl)methanone (reference example 14)

### Reactant: Ammonium thiocyanate

¹H NMR (300 MHz, [D6]DMSO): 5.55 (1 H, d, *J*=3.0Hz), 6.67-6.69 (2H, m), 6.74 (1 H, d, *J*=7.7Hz), 6.98 (1 H, d, *J*=8.5Hz), 7.13-7.16 (1 H, m), 7.37-7.41 (2H, m), 9.31 (1H, brs), 9.55 (1H, s), 10.78 (1H, brs);
¹³C NMR (75 MHz, [D6]DMSO): δ (ppm) = 56.7, 113.7, 115.0, 115.5, 117.0, 117.5, 124.4, 130.1, 130.5, 131.7, 134.2, 146.0, 158.4, 175.3;
ESI-HRMS *m*/*z*: calcd for C₁₄H₁₂BrN₂OS (MH⁺) 334.98482, found 334.98416;
m.p.= 115-117°C.

### Example 11: 6-Chloro-3,4-dihydro-4-phenylquinazoline-2(1H)-thione.

### Method: General procedure C d)

### Starting compound: (2-Amino-5-chlorophenyl)(phenyl)methanone

### Reactant: Ammonium thiocyanate

¹H NMR (300 MHz, [D6]DMSO): δ (ppm) = 5.66 (1 H, d, *J*=3.3), 7.05 (1 H, d, *J*=8.4Hz), 7.26-7.41 (6H, m), 9.36 (1H, s), 10.8(1H, s);
¹³C NMR (75 MHz, [D6]DMSO): δ (ppm) = 56.9, 116.7, 124.0, 126.9, 127.3, 128.5, 129.0, 129.5, 133.9, 144.5, 175.4;
ESI-HRMS *m*/*z*: calcd for C₁₄H₁₂ClN₂S (MH⁺) 275.04042, found 275.04086;
m.p.= 225-227°C.

### Example 12: 6-Chloro-4-(2'-fluorophenyl)-3,4-dihydroquinazoline-2(1H)-thione. Method: General procedure C d)

### Starting compound: (2-amino-5-bromophenyl)(2'-fluorophenyl)methanone

### Reactant: Ammonium thiocyanate

¹H NMR (300 MHz, [D6]DMSO): δ (ppm) = 5.92 (1 H, *J*=2.8Hz), 7.03-7.07 (2H, m), 7.22-7.43 (5H, m), 9.23 (1 H, brs), 10.88 (1 H, brs);
¹³C NMR (75 MHz, [D6]DMSO): δ (ppm) = 51.5,115.9,116.1 (d, *J*=5.7Hz), 121.8, 124.9 (d, *J*=3.4Hz), 126.2, 126.5, 128.5, 128.7 (d, *J*=4.0Hz), 130.2 (d, *J*=8.0Hz), 130.5 (d, *J=*13.1Hz), 133.2, 159.0 (d, *J*=246.8), 174.5;
ESI-HRMS *m*/*z*: calcd for C₁₄H₁₁ClFN₂OS (MH⁺) 293.03100, found 293.03101;
m.p.= 279-281°C

### Example 13: 3,4-Dihydro-4-phenylquinazoline-2(1H)-thione.

### Method: General procedure C d)

### Starting compound: 2-Aminobenzophenone

### Reactant: Ammonium thiocyanate

Physical and spectroscopic data for this compound are identical with those already published in D. Xu, P. G. Kucerovy, K. Prasad, O. Repic, *Tetrahedron Asymmetry* 1996, 7, 747-754.

### Assays and pharmacological data

### Expression and purification of Eg5₄₈₇H:

A sequence of *Xenopus laevis* Eg5 corresponding to amino acids 1 to 487 was amplified by polymerase chain reaction (PCR) from a full-length clone of Eg5 and was cloned into pQE70 (SphI/BglII) introducing a C-terminal (Gly)₅(His)₆ tag. The protein was expressed in *E. coli* M15(pREP4) for 5 h at 18 °C in LB containing 1 mM isopropylthio-β-D-galactopyranoside (IPTG), 100 µg/ml ampicillin, 25 µg/ml kanamycin, and 2% (v/v) ethanol. The harvested cells were lysed in buffer A (50 mM KPᵢ, 100 mM NaCl, 0.1 mM MgATP protease inhibitors (Roche), pH 7.0) with 5 mM mercaptoethanol (ME). The clarified lysate was loaded onto a HIS-Select Cobalt Affi Gel column (Sigma), washed with buffer A supplemented with 10 mM imidazole, 2 mM ME, 10 % (w/v) glycerol, and eluted with 150 mM imidazole, 50 mM KCI, 10 % (w/v) glycerol, 10 mM ME, pH 7.3. The buffer was exchanged to 50 mM 3-(N-morpholino)propanesulfonic acid/KOH, 50 mM KCI, 1 mM MgCl₂, 1 mM EGTA, 10 mM ME, 20 % (w/v) glycerol, pH 7.0 by dialysis. The protein concentration was adjusted to 2 mg/ml (measured by a Bradford assay using bovine serum albumin (BSA) as standard). Eg5₄₈₇H was frozen in liquid ethane and stored in liquid nitrogen.

### Microtubule polymerization in vitro:

A mixture of 50 µl purified pig brain tubulin , 50 µl BRB80 (80 mM PIPES pH 6.8, 1 mM MgCl₂ and 1 mM EGTA), 42.5 mg glycerol and 1.25 µl 100 mM GTP was incubated at 37 °C for 30 minutes. Polymerized microtubules were then stabilized by addition of 1 µl 10 mM paclitaxel (Sigma) in DMSO and incubated at 37 °C for another 20 minutes. The suspension was centrifuged at 70000 rpm at 30 °C for 30 minutes in a TLA100 rotor (Beckman). The resulting microtubule pellet was resuspended in 60 µl assay buffer (10 mM imidazole/acetate, 5 mM K-acetate, 2 mM EGTA and 4 mM Mg-acetate, pH 6.8.) supplemented with 20 µM paclitaxel at 37 °C. This microtubule suspension was stored at room temperature. The final concentration was 80 µM polymerized tubulin as determined by Bradford using BSA as a standard.

### IC₅₀ determination:

To determine the compound concentration that inhibits the microtubule-stimulated ATPase activity of Eg5 by 50% (IC₅₀) the EnzChek® Phosphate Assay (Molecular Probes) was used. Final concentrations were 40 nM Eg5₄₈₇H, 4 µM microtubules (tubulin dimer concentration as determined by Bradford using BSA as standard), and 1 mM MgATP. The ATPase activity was measured as an increase of absorbance at 365 nm within 2 h at room temperature. 1 % (v/v) inhibitor in DMSO at different concentrations (or 1 % (v/v) DMSO as a control) was added at the start of the experiment. At least 4 measurements were performed per condition and the results were then averaged. The microtubule-stimulated activity of Eg5₄₈₇H was obtained by subtracting background activity (measured with microtubules only in the absence of Eg5₄₈₇H) from measurements containing Eg5₄₈₇H and microtubules. The IC₅₀ was determined by assuming non-competitive enzyme inhibition and fitting the theoretically expected dependence of the enzym activity on the inhibitor concentration to the experimental microtubule-stimulated ATPase activities. The equation used for the fit was v = vₘₐₓ * IC₅₀ / ([inhibitor] + IC₅₀) + vᵣₑₛ with v being the measured relative ATPase rate, vₘₐₓ being the maximum ATPase rate and vᵣₑₛ being a residual activity at high inhibitor concentration.

The IC₅₀ values for the compounds of examples 1 to 3 were measured. The compounds of examples 1 and 3 had an IC₅₀ of 2 µM, i.e. were more than ten times more potent inhibitors of Eg5 as compared to monastrol (that has an IC₅₀ of 30 µM). The compound of example 2 had an IC₅₀ of 0.2 µM and thus was even more than 100 times more potent than monastrol. All three compounds inhibited Eg5 activity completely at concentrations well above the IC₅₀. In control experiments, no inhibition of conventional kinesin, Xenopus kinesin-like protein 1 (Xklp1) or Xenopus Kid (XKid) was observed with these three compounds indicating that inhibiton of Eg5 is specific.

### Cell culture and FACS analysis:

Then the effect of said compounds on exponentially growing human tissue culture cells was tested.

Hela cells were cultured in DMEM medium supplemented with 10 % (v/v) FCS to 60 % confluency. The culture medium was then exchanged by fresh medium containing different inhibitor or DMSO and the cells placed at 37 °C in 5 % CO₂ for 18 h. The final volume of inhibitor dissolved in DMSO in the medium was always equal to or less than 0.1 % (v/v)For wash out experiments, cells were treated as described above. After 18 h cells were washed with phosphate buffered saline (PBS), covered with fresh medium and cultured for another 24 h at 37 °C in 5 % CO₂.

Cells were visualized by epifluorescence (Zeiss Axioscope 2 equipped with a CCD camera) or by confocal microscopy (Zeiss LSM 510) before and after inhibitor treatment and after compound wash-out. For epi-fluorescence microscopy, cells were fixed with methanol at -20°C before processing for immunofluorescence. Tubulin was stained with a primary mouse monoclonal anti-α tubulin antibody DM1A (Sigma) and a secondary rabbit polyclonal Alexa 488-conjugated anti-mouse antibody (Molecular Probes). DNA was stained with Hoechst 33342. For confocal microscopy, cells were with XX% glutaraldehyde and processed for immunofluorescence. DNA was stained with a primary polyclonal rabbit anti-phospho-histone H3 antibody (Upstate Biotechnology) followed by a secondary polyclonal Alexa 546 conjugated anti-rabbit antibody. Microtubules were stained as described above.

HeLa cells were incubated for 18 hours in medium supplemented with various concentrations of either of the three compounds, monastrol or DMSO. Cells were then fixed and analyzed by FACS and immunofluorescence.

For FACS analysis, cells were scraped off the culture plate after inhibitor treatment or after wash-out and collected by centrifugation in a Heraeus Megafuge 1.0R at 1200 rpm for 5 min. The cell pellet was washed once with 5 ml PBS and then resuspended in 0.5 ml PBS. The cells were fixed by addition of 4 ml 70 % (v/v) ethanol and incubation overnight at -20 °C. Fixed cells were centrifuges at 1200 rpm for 5 min, washed with 5 ml PBS, and resuspended in 1ml PI solution (10 µg/ml propidium iodide, 100 µg/ml RNAse A, 0.05 % Triton X100 in PBS). After 30 min at 37 °C in the dark, cells were analyzed by fluorescence-activated cell sorting (FACS).

FACS analysis showed that monastrol blocked efficiently cells in G2/M (74%) at 100 µM. A lower concentration (10 µM) promoted only a very mild increase in G2/M cells (31 % versus 23% in the control). This is in agreement with previous reports. The three compounds tested also blocked cells in G2/M but did so at considerably lower concentrations than monastrol. Indeed 10 µM the compound of example 1 or example 3 had the same effect as 100 µM monastrol, promoting the accumulation of 77% of cells in G2/M. Strikingly, only 1 µM of the compound of example 2 were sufficient for the accumulation of 75% of the cells in G2/M cells. No cell cycle distribution abnormalities were observed in cells treated with 0.1 µM or the compound of example 2. Therefore, the effect of intermediate concentrations of this compound was investigated and it was found that 0.5 µM of said compound resulted in the accumulation of 47% of cells in G2/M. The concentrations required for mitotic arrest were similar to those required for the inhibition of Eg5 activity in vitro. This suggests a mechanism for cell cycle arrest mediated specifically through Eg5 inhibition.

One useful characteristic of monastrol is that is non-toxic for the cells when applied transiently; once washed out cells resume mitosis. It was therefore tested whether the three new compounds also shared these properties. HeLa cells were incubated with various concentrations of the different compounds as in the previous experiment. After 18 hours the compounds were washed out and the cells incubated in fresh medium for 24 hours before analysis. In all the conditions tested, cells reentered the cell cycle, indicating that like monastrol the three new compounds can be washed out from the cells and are non toxic.

We then examined the morphology of the cells treated with the different compounds by immonufluorescence with an anti-tubulin antibody and Hoechst. The same phenotype was observed for monastrol and the three compounds: cells were arrested in mitosis with a radial arrangement of microtubules and chromosomes.

### Xenopus egg extracts:

Finally, it was tested whether the compounds of examples 1 to 3 would generate the same effect in Xenopus egg extracts as in the human tissue culture cells.

Cytostatic factor (CSF)-arrested *Xenopus laevis* egg extracts were prepared as described previously. Spindle assembly experiments were performed in cycled extracts with sperm nuclei and tetramethylrhodamine labelled tubulin as described. 1 % (v/v) (or less) inhibitor dissolved in DMSO was added to the extract prior to spindle assembly. As control 1% DMSO was added to the extract. Spindles were fixed and centrifuged onto coverslips. The DNA was stained with DAPI. Structures were then visualized by epi-fluorescence microscopy (Zeiss Axiovert 135 equipped with a Roper CoolSnap HQ camera). Spindles and aberrant microtubule structures were then counted.

100 µM monastrol, 10 µM of the compound of example 1 or 3, or 1 µM of the compound of example 2 were added to a Xenopus egg extract. Spindle assembly was monitored in these and we found that all compounds inhibited bipolar spindle formation and promoted the formation of circular figures formed by radial arrangements of microtubules and chromosomes as was previously described for monastrol and as we observed in tissue culture cells.

## Claims

1. Quinazoline derivatives of formula (I) 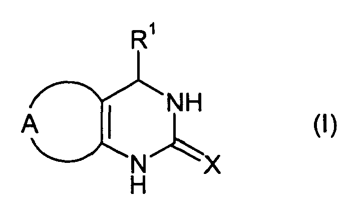 in which
R¹ denotes phenyl or six-membered heteroaryl, optionally substituted with one to five identical or different substituents selected from the group consisting of halogen, cyano, nitro, hydroxyl, mercapto, C₁-C₆-alkyl, C,-C₆-alkoxy, C₁-C₆-alkylthio, C₁-C₆-haloalkyl, C₁-C₆-haloalkoxy, carboxyl, C₁-C₆-alkylcarbonyl, C₁-C₆-alkoxycarbonyl and C₁-C₆-alkylcarbonyloxy;
X denotes S or O;
A together with the carbon atoms to which it is bonded forms a five-, six- or seven-membered, partially unsaturated or aromatic, homocyclic or heterocyclic ring which may carry up to ten identical or different substituents selected from the group consisting of halogen, cyano, nitro, hydroxyl, oxo, mercapto, thio, amino, C₁-C₆-alkyl, C₁-C₆-alkoxy, C₁-C₆₋alkylthio, C₁-C₆-alkylamino, di-(C₁-C₆-alkyl)amino, C₁-C₆-haloalkyl, C₁₋C₆-haloalkoxy, carboxyl, carbamoyl, C₁-C₆-alkylcarbonyl, C₁-C₆₋alkoxycarbonyl, C₁-C₆-alkylcarbonyloxy, C₁-C₆-alkylaminocarbonyl, di-(C₁-C₆-alkyl)amincarbonyl, C₁-C₆-alkylsulfinyl, C₁-C₆-alkylsulfonyl, hydroxysulfonyl, aminosulfonyl, C₁-C₆-alkylaminosulfonyl and di-(C₁-C₆₋alkyl)aminosulfonyl,
or a pharmaceutically acceptable salt thereof.

2. The quinazoline derivative or salt thereof according to claim 1, wherein in formula (I) R¹ denotes a group of formula (II): 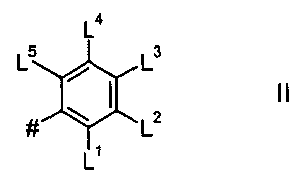 wherein L¹ to L⁵ independently denote halogen, cyano, nitro, hydroxyl, mercapto, C₁-C₆-alkyl, C₁-C₆-alkoxy, C₁-C₆-alkylthio, C₁-C₆-haloalkyl, C₁-C₆-haloalkoxy, carboxyl C₁-C₆-alkylcarbonyl, C₁-C₆-alkoxycarbonyl, C₁-C₆-alkylcarbonyloxy, and # marks the bond to the quinazoline nucleus, wherein at least one of L¹ to L⁵ is not hydrogen.

3. The quinazoline derivative or salt thereof according to claim 1 or 2, wherein in formula (I) R¹ is a 3-hydroxyphenyl group.

4. The quinazoline derivative or salt thereof according to any one of claims 1 to 3, wherein in formula (I) X is S.

5. The quinazoline derivative or salt thereof according to any one of daims 1 to 4, wherein in formula (I) A together with the carbon atoms to which it is bonded forms a six-membered, partially unsaturated or aromatic homocyclic ring which may up to eight identical or different substituents selected from the group as defined in claim (I).

6. The quinazoline derivative or salt thereof according to claim 5, which is a quinazoline derivative of formula (Ia): 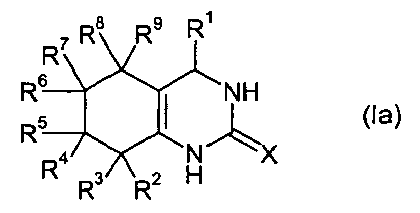 wherein R¹ and X are as defined in any one of claims 1 to 5, and R², R³, R⁴, R⁵, R⁶, R⁷, R⁸, R⁹ independently denote hydrogen, halogen, cyano, nitro, hydroxyl, oxo, mercapto, thio, amino, C₁-C₆-alkyl, C₁-C₆-alkoxy, C₁-C₆-alkylthio, C₁-C₆₋alkylamino, di-(C₁-C₆-alkyl)amino, C₁-C₆-haloalkyl, C₁-C₆-haloalkoxy, carboxyl, carbamoyl, C₁-C₆-alkylcarbonyl, C₁-C₆-alkoxycarbonyl, C₁-C₆-alkylcarbonyloxy, C₁-C₆-alkylaminocarbonyl, di-(C₁-C₆-alkyl)amincarbonyl, C₁-C₆-alkylsulfinyl, C₁₋C₆-alkylsulfonyl, hydroxysulfonyl, aminosulfonyl, C₁-C₆-alkylaminosulfonyl or di-(C₁-C₆-alkyl)aminosulfonyl, or R³ together with R⁵, R⁵ together with R⁷ and/or R⁷ together with R⁹ form a bond, or R² together with R³, R⁴ together with R⁵, R⁶ together with R⁷, and/or R⁸ together with R⁹ form an oxo or thio group, or the salt thereof.

7. The quinazoline derivative or salt thereof according to claim 6, wherein in formula (Ia) R⁸ and R⁹, together with the carbon atom to which they are bonded, form a carbonyl group and R², R³, R⁴, R⁵, R⁶, R⁷ are as defined in claim 6.

8. The quinazoline derivative or salt thereof according to claim 6, wherein in formula (Ia) R⁸ is hydrogen, R⁹ is hydroxyl, and R², R³, R⁴, R⁵, R⁶, R⁷ are as defined in claim 6.

9. The quinazoline derivative or salt thereof according to claim 5, which is a quinazoline derivative of formula (Id): 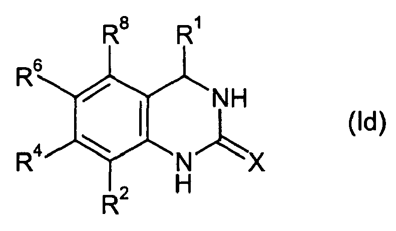 wherein R¹ and X are as defined in any one of claims 1 to 5, and R², R⁴, R⁶, R⁸ independently denote hydrogen, halogen, cyano, nitro, hydroxyl, mercapto, amino, C₁-C₆-alkyl, C₁-C₆-alkoxy, C₁-C₆-alkylthio, C₁-C₆-alkylamino, di-(C₁-C₆₋alkyl)amino, C₁-C₆-haloalkyl, C₁-C₆-haloalkoxy, carboxyl, carbamoyl, C₁-C₆₋alkylcarbonyl, C₁-C₆-alkoxycarbonyl, C₁-C₆-alkylcarbonyloxy, C₁-C₆₋alkylaminocarbonyl, di-(C₁-C₆-alkyl)amincarbonyl, C₁-C₆-alkylsulfinyl, C₁-C₆₋alkylsulfonyl, hydroxysulfonyl, aminosulfonyl, C₁-C₆-alkylaminosulfonyl or di-(C₁₋C₆-alkyl)aminosulfonyl,
or the salt thereof.

10. The quinazoline derivative or salt thereof of claim 1, which is: 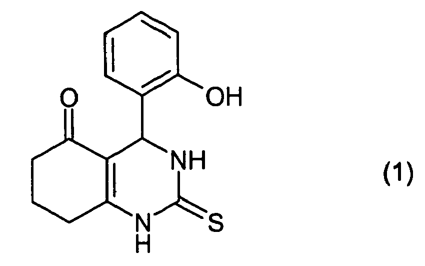 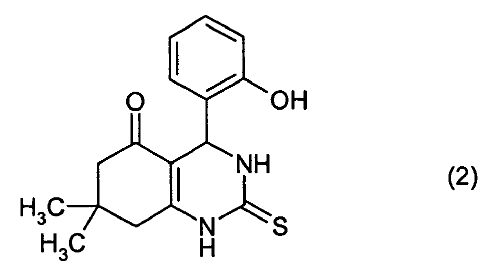 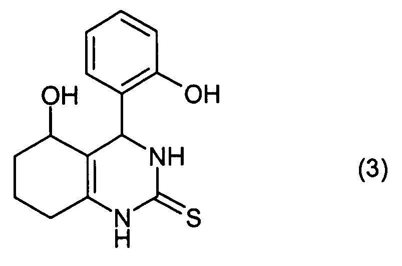 or the salt thereof.

11. A quinazoline derivative or salt thereof of any one of claims 1 to 10 for use in therapy.

12. A pharmaceutical composition comprising one or more than one quinazoline derivative or salt thereof of any one of claims 1 to 10 and optionally one or more pharmaceutcallly acceptable excipients.

13. The use of a quinazoline derivative or salt thereof of any one of claims 1 to 10 for the treatment of cancer.
